(19) Europäisches Patentamt
European Patent Office
Office européen des brevets

(11) EP 2 398 818 B1

(12) # FASCICULE DE BREVET EUROPEEN

(45) Date de publication et mention de la délivrance du brevet:
**29.08.2018 Bulletin 2018/35**

(21) Numéro de dépôt: **10709893.1**

(22) Date de dépôt: **19.02.2010**

(51) Int Cl.:
***C07K 1/22*** (2006.01) ***C07K 14/745*** (2006.01)
***C07K 14/755*** (2006.01) ***C07K 14/765*** (2006.01)
***C07K 14/78*** (2006.01) ***C07K 14/81*** (2006.01)
***C07K 16/00*** (2006.01) ***C12N 9/64*** (2006.01)
***C12N 15/115*** (2010.01)

(86) Numéro de dépôt international:
**PCT/FR2010/050294**

(87) Numéro de publication internationale:
**WO 2010/094901 (26.08.2010 Gazette 2010/34)**

(54) **MOYENS POUR LA PURIFICATION D'UNE PROTEINE DU PLASMA SANGUIN, ET PROCEDES POUR SA MISE EN OEUVRE**

MITTEL ZUR AUFREINIGUNG EINES PROTEINS AUS BLUTPLASMA UND VERFAHREN ZU DESSEN EINSATZ

MEANS FOR PURIFYING A PROTEIN OF BLOOD PLASMA AND METHODS FOR IMPLEMENTING SAME

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **19.02.2009 FR 0951083**

(43) Date de publication de la demande:
**28.12.2011 Bulletin 2011/52**

(73) Titulaire: **Laboratoire Français du Fractionnement et des Biotechnologies**
**91940 Les Ulis (FR)**

(72) Inventeurs:
- **PERRET, Gérald**
**F-94600 Choisy Le Roi (FR)**
- **NOGRE, Michel**
**F-92170 Vanves (FR)**

(74) Mandataire: **Verhaeghe Bect, Elodie**
**Cabinet Becker & Associés**
**25, rue Louis le Grand**
**75002 Paris (FR)**

(56) Documents cités:
**WO-A1-2007/058592 WO-A2-2006/023831**
**WO-A2-2008/099077 US-A- 5 986 062**
**US-A- 6 117 996**

- **QIANG ZHAO, XING-FANG LI, YUANHUA SHAO, X. CHRIS LE: "Aptamer-Based Affinity Chromatography Assays for Thrombin" ANALYTICAL CHEMISTRY, vol. 80, no. 19, 1 octobre 2008 (2008-10-01), pages 7586-7593, XP002557962 cité dans la demande**
- **LIU XUEMEI ET AL: "RNA aptamers specific for bovine thrombin" JOURNAL OF MOLECULAR RECOGNITION, HEYDEN & SON LTD., LONDON, GB, vol. 16, no. 1, 1 janvier 2003 (2003-01-01), pages 23-27, XP002479553 ISSN: 0952-3499**
- **ROMIG T S ET AL: "Aptamer affinity chromatography: - combinatorial chemistry applied to protein purification", JOURNAL OF CHROMATOGRAPHY B: BIOMEDICAL SCIENCES AND APPLICATIONS, ELSEVIER SCIENCE PUBLISHERS, NL, vol. 731, no. 2, 27 August 1999 (1999-08-27), pages 275-284, XP004179577, ISSN: 0378-4347, DOI: 10.1016/S0378-4347(99)00243-1**
- **ZHAO Q ET AL: "Aptamer-Modified Monolithic Capillary Chromatography for Protein Separation and Detection", ANALYTICAL CHEMISTRY, AMERICAN CHEMICAL SOCIETY, US, vol. 80, no. 10, 15 May 2008 (2008-05-15), pages 3915-3920, XP002574911, ISSN: 0003-2700, DOI: 10.1021/AC702567X [retrieved on 2008-03-26]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- MIYAKAWA SHIN ET AL: "Structural and molecular basis for hyperspecificity of RNA aptamer to human immunoglobulin G", RNA, COLD SPRING HARBOR LABORATORY PRESS, US, vol. 14, no. 6, 1 June 2008 (2008-06-01), pages 1154-1163, XP002532239, ISSN: 1355-8382, DOI: 10.1261/RNA.1005808
- MICHAUD M ET AL: "A DNA aptamer as a new target-specific chiral selector for HPLC", JOURNAL OF THE AMERICAN CHEMICAL SOCIETY, AMERICAN CHEMICAL SOCIETY, US, vol. 125, no. 28, 16 July 2003 (2003-07-16), pages 8672-8679, XP002364016, ISSN: 0002-7863, DOI: 10.1021/JA034483T

SEQUENCE LISTING

<110> LFB BIOTECHNOLOGIES

<120> Moyens pour la purification d\222une protéine du plasma sanguin, et procédés pour sa mise en oeuvre

<130> W515PCT

<160> 101

<170> PatentIn version 3.3

<210> 1
<211> 18
<212> DNA
<213> artificial sequence

<220>
<223> aptamer

<400> 1
gggagatagc cacgacct 18

<210> 2
<211> 18
<212> DNA
<213> artificial sequence

<220>
<223> aptamer

<400> 2
tccaggctgt gcgaaagc 18

<210> 3
<211> 44
<212> DNA
<213> artificial sequence

<220>
<223> aptamer

<400> 3
ccgcacacca cgcgcattag cccgcgcaca cgacttgaag tagc 44

<210> 4
<211> 44
<212> DNA
<213> artificial sequence

<220>
<223> aptamer

<400> 4
ccgcacacca cgcgcattag cccgcgcaca cgacttgaag tagc 44

<210> 5
<211> 43
<212> DNA
<213> artificial sequence

<220>
<223> aptamer

<400> 5
cgcacaccac gcgcattagc ccgcgcacac gacttgaagt agc 43

<210> 6
<211> **44**
<212> DNA
<213> artificial sequence

<220>
<223> aptamer

<400> 6
ccgcacacca cgcgcatgaa cccgcgcaca cgacttgaag tagc 44

<210> 7
<211> **44**
<212> DNA
<213> artificial sequence

<220>
<223> aptamer

<400> 7
ccgcacacca cgcgcatgaa cccgcgcaca cgacttgaag tagc 44

<210> 8
<211> 44
<212> DNA
<213> artificial sequence

<220>
<223> aptamer

<400> 8
ccgcacacca cgcgcatgaa cccgcgcaca cgacttgaag tagc 44

<210> 9
<211> 44
<212> DNA
<213> artificial sequence

<220>
<223> aptamer

<400> 9
ccgcacacca cgcgcatgaa cccgcgcaca cgacttgaag tagc 44

<210> 10
<211> 44
<212> DNA
<213> artificial sequence

<220>
<223> aptamer

<400> 10
acgcacacca cgcgcatgaa cccgcgcaca cgacttgaag tagc 44

<210> 11
<211> 44
<212> DNA
<213> artificial sequence

<220>
<223> aptamer

<400> 11
ccgcacacca cgcgcatgag cccgcgcaca cgacttgaag tagc 44

<210> 12
<211> 44
<212> DNA
<213> artificial sequence

<220>
<223> aptamer

<400> 12
ccgcacacca cgcgcatgag cccgcgcaca cgacttgaag tagc 44

<210> 13
<211> 44
<212> DNA
<213> artificial sequence

<220>
<223> aptamer

<400> 13
ccgcacacca cgcgcatgag cccgcgcaca cgacttgaag tagc 44

<210> 14
<211> 45
<212> DNA
<213> artificial sequence

<220>
<223> aptamer

<400> 14
gccgcacacc acgcgcatga gcccgcgcac acgacttgaa gtagc 45

<210> 15
<211> 43
<212> DNA
<213> artificial sequence

<220>
<223> aptamer

<400> 15
cgcacaccac gcgcatgagc ccgcgcacac gacttgaagt agc 43

<210> 16
<211> 44
<212> DNA
<213> artificial sequence

<220>
<223> aptamer

<400> 16
tcgcacacca cgcgcatgag cccgcgcaca cgacttgaag tagc **44**

<210> 17
<211> 44
<212> DNA
<213> artificial sequence

<220>
<223> aptamer

<400> 17
acgcacacca cgcgcatgag cccgcgcaca cgacttgaag tagc 44

<210> 18
<211> 44
<212> DNA
<213> artificial sequence

<220>
<223> aptamer

<400> 18
acgcacacca cgcgcatgag cccgcgcaca cgacttgaag tagc 44

<210> 19
<211> 43
<212> DNA
<213> artificial sequence

<220>
<223> aptamer

<220>
<221> misc_feature
<222> (19)..(19)
<223> n is a, c, g, or t

<400> 19

cgcacaccac gcgcatganc ccgcgcacac gacttgaagt agc 43

<210> 20
<211> 44
<212> DNA
<213> artificial sequence

<220>
<223> aptamer

<400> 20
ccgcacacca cgcgcataat cccgcgcaca cgacttgaag tagc 44

<210> 21
<211> 44
<212> DNA
<213> artificial sequence

<220>
<223> aptamer

<400> 21
ccgcacacca cgcgcatgac cccgcgcaca cgacttgaag tagc 44

<210> 22
<211> 44
<212> DNA
<213> artificial sequence

<220>
<223> aptamer

<400> 22
ccgcacacca cgcgcatgac cccgcgcaca cgacttgaag tagc 44

<210> 23
<211> 43
<212> DNA
<213> artificial sequence

<220>
<223> aptamer

<400> 23
cgcacaccac gcgcgtgacc ccgcgcacac gacttgaagt agc 43

<210> 24
<211> 44
<212> DNA
<213> artificial sequence

<220>
<223> aptamer

<400> 24
ccgcacacca cgcgcattaa cccgcgcaca cggcttggag tagc 44

<210> 25

<211> 44
<212> DNA
<213> artificial sequence

<220>
<223> aptamer

<400> 25
tcgcacacta cgcgcatgaa cccgcgcaca cgacttgaag tagc 44

<210> 26
<211> 44
<212> DNA
<213> artificial sequence

<220>
<223> aptamer

<400> 26
tcgcacacta cgcgcatgaa cccgcgcaca cgacttgaag tagc 44

<210> 27
<211> 43
<212> DNA
<213> artificial sequence

<220>
<223> aptamer

<400> 27
cgcacactac gcgcatgaac ccgcgcacac gacttgaagt agc 43

<210> 28
<211> 45
<212> DNA
<213> artificial sequence

<220>
<223> aptamer

<400> 28
gccgcacact acgcgcatga gcccgcgcac acgacttgaa gtagc 45

<210> 29
<211> 44
<212> DNA
<213> artificial sequence

<220>
<223> aptamer

<400> 29
tcgcacacta cgcgcatgag cccgcgcaca cgacttgaag tagc 44

<210> 30
<211> 44
<212> DNA
<213> artificial sequence

<220>
<223> aptamer

<400> 30
ccgcacacta cgcgcatgat cccgcgcaca cgacttgaag tagc 44

<210> 31
<211> 44
<212> DNA
<213> artificial sequence

<220>
<223> aptamer

<400> 31
ccgcacacta cgcgcatgat cccgcgcaca cgacttgaag tagc 44

<210> 32
<211> 44
<212> DNA
<213> artificial sequence

<220>
<223> aptamer

<400> 32
ctgcacacta cgcgcatgat cccgcgcaca cgacttgaag tagc 44

<210> 33
<211> 44
<212> DNA
<213> artificial sequence

<220>
<223> aptamer

<400> 33
ccgcacgcta cgcgcatgaa cccgcgcaca cgacttgaag tagc 44

<210> 34
<211> 44
<212> DNA
<213> artificial sequence

<220>
<223> aptamer

<400> 34
ccgcacacta cgcgcacgaa cccgcgcaca cgacttgaag tagc 44

<210> 35
<211> 44
<212> DNA
<213> artificial sequence

<220>
<223> aptamer

<400> 35
tcgcacacta cgcgcatgac cccgcgcaca cgacttgaag tagc 44

<210> 36
<211> 43
<212> DNA
<213> artificial sequence

<220>
<223> aptamer

<400> 36
agcacaccac gcgcataaac ccgcgcacac gacttgaagt agc 43

<210> 37
<211> 44
<212> DNA
<213> artificial sequence

<220>
<223> aptamer

<400> 37
gagcacacca cgcgcatgaa cccgcgcaca cgacttgaag tagc 44

<210> 38
<211> 46
<212> DNA
<213> artificial sequence

<220>
<223> aptamer

<400> 38
gctagcacac cacgcgcatg aacccgcgca cacgacttga agtacc 46

<210> 39
<211> 44
<212> DNA
<213> artificial sequence

<220>
<223> aptamer

<400> 39
acgcacacca cgcgcatgaa cccgcgcaca tgacttgaag tagc 44

<210> 40
<211> 44
<212> DNA
<213> artificial sequence

<220>
<223> aptamer

<400> 40
ccgcacacca cgcgcattag cccgcgcaca cgacttgaag ttaa 44

<210> 41
<211> 44
<212> DNA
<213> artificial sequence

<220>
<223> aptamer

<400> 41
ccggagacgc gcagctccta tacatgcgca catgacttga agtc 44

<210> 42
<211> 44
<212> DNA
<213> artificial sequence

<220>
<223> aptamer

<400> 42
ccggagacgc gcagctccta tacatgcgca catgacttga agtc 44

<210> 43
<211> 44
<212> DNA
<213> artificial sequence

<220>
<223> aptamer

<400> 43
cttgagacgc gcatttgcta tacatgcgca cgtgacttga agtc 44

<210> 44
<211> 44
<212> DNA
<213> artificial sequence

<220>
<223> aptamer

<400> 44
cttgagacgc gcatttgcta tacatgcgca cgtgacttga agtc 44

<210> 45
<211> 43
<212> DNA
<213> artificial sequence

<220>
<223> aptamer

<220>
<221> misc_feature
<222> (13)..(13)
<223> n is a, c, g, or t

<400> 45

ctgagacgcg canttgctat acatgcgcac atgacttgaa gtc 43

<210> 46
<211> 46
<212> DNA
<213> artificial sequence

<220>
<223> aptamer

<400> 46
ctggagacgc gcagttgctg tacatgcgca catgacttga agtagc 46

<210> 47
<211> 44
<212> DNA
<213> artificial sequence

<220>
<223> aptamer

<400> 47
gtggagacgc gcatttgctg tacatgcgca catgactaga agtc 44

<210> 48
<211> 44
<212> DNA
<213> artificial sequence

<220>
<223> aptamer

<400> 48
caggagacgc gcatttgttg tacatgcgca catgactaga agtc 44

<210> 49
<211> 44
<212> DNA
<213> artificial sequence

<220>
<223> aptamer

<400> 49
ctggagacgc gcagttgttg tacacgcgca catgactaga agtc 44

<210> 50
<211> 44
<212> DNA
<213> artificial sequence

<220>
<223> aptamer

<400> 50
ctggagacgc gcagttgcta cacacgcgca catgactaga agtc 44

<210> 51

<211> 44
<212> DNA
<213> artificial sequence

<220>
<223> aptamer

<400> 51
ctggagacgc gcacttcctt tacatgcgca catgactaga agtc 44

<210> 52
<211> 44
<212> DNA
<213> artificial sequence

<220>
<223> aptamer

<400> 52
ctggagacgc gcagttgctt tacatgcgca catgactaga agtc 44

<210> 53
<211> 44
<212> DNA
<213> artificial sequence

<220>
<223> aptamer

<400> 53
ctggagacgc gcagttgctg tacatgcgca catgactaga agtc 44

<210> 54
<211> 44
<212> DNA
<213> artificial sequence

<220>
<223> aptamer

<400> 54
ctggagacgc gcagttgctg tacatgcgca catgactaga agtc 44

<210> 55
<211> 44
<212> DNA
<213> artificial sequence

<220>
<223> aptamer

<400> 55
ctggggacgc gcagttgctg tacatgcgca catgactaga agtc 44

<210> 56
<211> 44
<212> DNA
<213> artificial sequence

<220>
<223> aptamer

<400> 56
ctggagacgc gcagttacca tacgtgcgca catgactaga agtc 44

<210> 57
<211> 44
<212> DNA
<213> artificial sequence

<220>
<223> aptamer

<400> 57
ctggagacgc gcagttgctg tacgtgcgca catgactaga agtc 44

<210> 58
<211> 44
<212> DNA
<213> artificial sequence

<220>
<223> aptamer

<400> 58
agtctccatg ttgcgaaccg aatggtaagg ggatcgtaca ctac 44

<210> 59
<211> 44
<212> DNA
<213> artificial sequence

<220>
<223> aptamer

<400> 59
agtctccatg ttgcgaaccg aatggtaagg ggatcgtaca ctac 44

<210> 60
<211> 44
<212> DNA
<213> artificial sequence

<220>
<223> aptamer

<400> 60
agactccatg atgcgaaccg aatggtaagg gaatcgtaca atac 44

<210> 61
<211> 44
<212> DNA
<213> artificial sequence

<220>
<223> aptamer

<400> 61
agactccatg atgcgaaccg aatggtaagg gaatcgtaca atac 44

<210> 62
<211> 44
<212> DNA
<213> artificial sequence

<220>
<223> aptamer

<400> 62
agactccatt atgcgaaccg aatggtaagg gaatcgtaca atac 44

<210> 63
<211> 44
<212> DNA
<213> artificial sequence

<220>
<223> aptamer

<400> 63
agactccatg atgcgaaccg aatggtaagg gaatcgtaca gtac 44

<210> 64
<211> 44
<212> DNA
<213> artificial sequence

<220>
<223> aptamer

<400> 64
agactccatg atgcgaaccg aatggtaagg gaatcgtaca gtac 44

<210> 65
<211> 44
<212> DNA
<213> artificial sequence

<220>
<223> aptamer

<400> 65
agactccatg atgcgaaccg aatggtaagg gaatcgtaca atgc 44

<210> 66
<211> 44
<212> DNA
<213> artificial sequence

<220>
<223> aptamer

<400> 66
agactccatg atgcgaaccg aatggtaagg gaatcgtaca atgc 44

<210> 67
<211> 44
<212> DNA
<213> artificial sequence

<220>
<223> aptamer

<400> 67
agactccatg atgcgaaccg aatggtaagg gaatcgtaca atgc 44

<210> 68
<211> 44
<212> DNA
<213> artificial sequence

<220>
<223> aptamer

<400> 68
agactccatg atgcgaaccg aatggtaagg gaatcgtacg acac 44

<210> 69
<211> 44
<212> DNA
<213> artificial sequence

<220>
<223> aptamer

<400> 69
agactccatg gtgcgaaccg aatggtaagg gaatcgtaca acac 44

<210> 70
<211> 44
<212> DNA
<213> artificial sequence

<220>
<223> aptamer

<400> 70
tgactccatg atgcgaaccg aatggtaagg gaatcgtaca acac 44

<210> 71
<211> 44
<212> DNA
<213> artificial sequence

<220>
<223> aptamer

<400> 71
agactccatg atgcgaaccg aatggttagg gaatcgtaca atac 44

<210> 72
<211> 44
<212> DNA

<213> artificial sequence

<220>
<223> aptamer

<400> 72
agactccatg atgcgaaccg aatggttagg gaatcgtaca atac 44

<210> 73
<211> 44
<212> DNA
<213> artificial sequence

<220>
<223> aptamer

<400> 73
agactccatg atgcgaaccg aatggttagg gaatcgtaca **acac** 44

<210> 74
<211> 44
<212> DNA
<213> artificial sequence

<220>
<223> aptamer

<400> 74
agaccccatg atgcgaaccg aatggttagg gaatcgtaca gtac 44

<210> 75
<211> 44
<212> DNA
<213> artificial sequence

<220>
<223> aptamer

<400> 75
agaccccctg atgcgaaccg aatggttagg gaatcgtaca atac 44

<210> 76
<211> 44
<212> DNA
<213> artificial sequence

<220>
<223> aptamer

<400> 76
agactccatg atgcgaaccg aatggtaagg gaatcgcact gtac 44

<210> 77
<211> 44
<212> DNA
<213> artificial sequence

<220>

<223> aptamer

<400> 77
agactccaag atgcgaaccg aatggtaagg gaatcgtaca gtac 44

<210> 78
<211> 44
<212> DNA
<213> artificial sequence

<220>
<223> aptamer

<400> 78
agaccccgtg atgcgaaccg aatggtaagg gaatcgcaca atac 44

<210> 79
<211> 44
<212> DNA
<213> artificial sequence

<220>
<223> aptamer

<400> 79
agactccccg atgcgaaccg aatggtaagg gaatcgtacg atgc 44

<210> 80
<211> 44
<212> DNA
<213> artificial sequence

<220>
<223> aptamer

<400> 80
agaccccatg acgcgaaccg aatggtaagg gaatcgtaca atgc 44

<210> 81
<211> 44
<212> DNA
<213> artificial sequence

<220>
<223> aptamer

<400> 81
agaccccatg atgcgaaccg aatggtaagg gaatcgtact acac 44

<210> 82
<211> 44
<212> DNA
<213> artificial sequence

<220>
<223> aptamer

<400> 82

agaccccatg atgcgaaccg aatggtaagg ggatcgtaca accc 44

<210> 83
<211> 43
<212> DNA
<213> artificial sequence

<220>
<223> aptamer

<400> 83
agactccatg atgcgaaccg aatggtaagg gaatcataca ccc 43

<210> 84
<211> 44
<212> DNA
<213> artificial sequence

<220>
<223> aptamer

<400> 84
agactccatg atgctaaccg aatggttagg gaatcgtacg acgc 44

<210> 85
<211> 45
<212> DNA
<213> artificial sequence

<220>
<223> aptamer

<400> 85
atgcagccag ccgcagtgta agtgaatgca gacatggtct aagtg 45

<210> 86
<211> 81
<212> DNA
<213> artificial sequence

<220>
<223> aptamer

<400> 86

**gggagatagc cacgacctat gcagccagcc gcagtgtaag tgaatgcaga catggtctaa 60**

**gtgtccaggc tgtgcgaaag c 81**

<210> 87
<211> 43
<212> DNA
<213> artificial sequence

<220>
<223> aptamer

<400> 87
taagccgctg cgtattatag ctgaatgccc ctaatgggaa gtg 43

<210> 88
<211> 44
<212> DNA
<213> artificial sequence

<220>
<223> aptamer

<400> 88
catgccgctg cgtattatag ctgaatgccc cacaatggga agtg 44

<210> 89
<211> 44
<212> DNA
<213> artificial sequence

<220>
<223> aptamer

<400> 89
caagccgctg cgtattatag ctgaatgccc cataatggga agtg 44

<210> 90
<211> 43
<212> DNA
<213> artificial sequence

<220>
<223> aptamer

<400> 90
caagccgctg cgtatttagc tgaatgcccc ctaatgggaa gtg 43

<210> 91
<211> 44
<212> DNA
<213> artificial sequence

<220>
<223> aptamer

<400> 91
ccagccgctg cgtatcatag ctgaatgccc cataatggga agtg 44

<210> 92
<211> 43
<212> DNA
<213> artificial sequence

<220>
<223> aptamer

<400> 92
caagccgctg cgtaatatag ctgaatgccc cataatggaa gtg 43

<210> 93
<211> 44
<212> DNA
<213> artificial sequence

<220>
<223> aptamer

<400> 93
caagcctctg cgtattatag ctgaatgctc cttaatggta agtg 44

<210> 94
<211> 44
<212> DNA
<213> artificial sequence

<220>
<223> aptamer

<400> 94
caagccgctg cgtattatag ctgaatgctc cttaatggta agtg 44

<210> 95
<211> 44
<212> DNA
<213> artificial sequence

<220>
<223> aptamer

<400> 95
ccaggcgctg cgtatcatag ctgaatgctc cttaacggta agtg 44

<210> 96
<211> 44
<212> DNA
<213> artificial sequence

<220>
<223> aptamer

<400> 96
caagccgctg cgttttatag ctgaatgctc ctcaatggta agtg 44

<210> 97
<211> 44
<212> DNA
<213> artificial sequence

<220>
<223> aptamer

<400> 97
caagccgctg cgttttatag ctgaatgctc catcttggta agtg 44

<210> 98
<211> 44
<212> DNA

<213> artificial sequence

<220>
<223> aptamer

<220>
<221> misc_feature
<222> (18)..(18)
<223> n est a

<220>
<221> misc_feature
<222> (18)..(18)
<223> n est t

<220>
<221> misc_feature
<222> (18)..(18)
<223> n est g

<220>
<221> misc_feature
<222> (18)..(18)
<223> n est c

<400> 98
gtgcagccag ccgcagtnta agtgagctga ttgaagcatg aggg 44

<210> 99
<211> 45
<212> DNA
<213> artificial sequence

<220>
<223> aptamer

<400> 99
atgcagccag ccgcagtgta agtgaatgca gacatggtct aagtg 45

<210> 100
<211> 45
<212> DNA
<213> artificial sequence

<220>
<223> aptamer

<400> 100
ccagcagagc cgcagtttca gtgaatgcag atcatggtct aagtg 45

<210> 101
<211> 80
<212> DNA
<213> artificial sequence

<220>
<223> aptamer

<400> 101

```
gggagatagc cacgacctcg cacatgactt gaagttaaac gcgaattaca aacccagccc      60
cctccaggct gtgcgaaagc                                                  80
```

## Description

### DOMAINE DE L'INVENTION

La présente invention se rapporte au domaine de la purification des protéines du plasma sanguin, utilisables comme principes actifs de médicaments

### ART ANTERIEUR

Les protéines du plasma sanguin constituent depuis longtemps des principes actifs de médicaments. Parmi les protéines du plasma sanguin utilisées comme principes actifs de médicament, on peut citer notamment le facteur VII, le facteur VIII, la thrombine ou encore le facteur de Willebrand.

Jusqu'à une période récente, les protéines du plasma étaient exclusivement obtenues par purification à partir du plasma sanguin humain. Depuis quelques années, certaines protéines du plasma sont obtenues sous la forme de protéines recombinantes produites dans des fluides corporels de mammifères transgéniques, par exemple dans le lait de mammifères transgéniques.

Dans tous les cas, le matériel de départ contenant la protéine plasmatique à purifier consiste en un matériel de constitution complexe, dans lequel la protéine d'intérêt est présente en combinaison avec de très nombreuses substances, y compris des protéines, des lipides, des glucides, des acides aminés, des sels minéraux, des débris cellulaires et des déchets métaboliques comme l'urée, l'acide urique ou la bilirubine.

La mise au point d'un procédé de purification d'une protéine plasmatique est donc un travail complexe, compte tenu des caractéristiques sanitaires et réglementaires exigées pour la mise sur le marché d'un médicament à usage humain.

On comprend que, dans un médicament à usage humain ou vétérinaire, la protéine plasmatique utilisée comme principe actif doit être présente sous une forme hautement purifiée et ne pas être associée à des substances indésirables susceptibles d'être délétères pour l'organisme, y compris d'autres protéines plasmatiques, ou encore des produits de dégradation des protéines précitées.

On connait des procédés de purification de protéines plasmatiques variées, y compris des procédé de purification du Facteur VIII, de l'anti-thrombine-III, du plasminogène, du Facteur VII ou encore du facteur de Willebrand.

L'ensemble des procédés connus comprennent une succession d'étapes de séparation sélective basées sur des étapes de précipitation des protéines, de passage sur des supports de chromatographie suivi d'étapes d'élution séquentielle, d'étapes de filtration en profondeur, d'ultrafiltration ou encore d'étapes de concentration.

On précise que la mise au point de procédés de purification des protéines de la coagulation, que ces procédés soient entièrement nouveaux, ou bien que ces procédés soient des adaptations, même minimes, de procédés connus, nécessite de longues recherches et de nombreux contrôles de conformité des produits intermédiaires successifs, afin de s'assurer que le produit final sera obtenu avec une grande pureté, sous une forme non altérée, sensiblement exempt de substances indésirables et à la fois stérile et apyrogène. Notamment, pour les protéines plasmatiques de la coagulation ayant une activité enzymatique, comme par exemple les facteurs VII, VII et XI, il est essentiel que la protéine finale purifiée ne soit pas activée. Or, l'activation d'une protéine de la coagulation est susceptible d'être induite au cours de l'une quelconque des étapes de purification, y compris durant les étapes de chromatographie, si des conditions de consigne prédéterminées, par exemple qualité des filtres ou du support chromatographique utilisés, concentration saline ou encore température, ne sont pas respectées.

Ce qui précède explique, au moins en partie, pourquoi les procédés connus ne comprennent pas d'étape de chromatographie d'affinité, basée sur le principe d'immobilisation spécifique de la protéine plasmatique d'intérêt sur un ligand greffé sur le support chromatograhique, puis récupération de la protéine purifiée dans l'éluat de chromatographie.

L'absence d'étape de purification par chromatographie d'affinité, dans les procédés de purification de protéines d'intérêt thérapeutique est expliquée également par les inconvénients de cette technique, dans laquelle on observe le détachement d'une partie des molécules de ligand greffées sur le support d'affinité, qui sont retrouvées associées à la protéine thérapeutique purifiée dans le volume de l'éluat. On comprend que la présence, dans un médicament comprenant une protéine plasmatique purifiée, de substances constitutives d'un support de chromatographie, qui sont éventuellement délétères pour l'organisme, peut mettre en jeu la santé du patient et est proscrite par la réglementation médicale.

Romig et al. (J. Chromatogr. 1999, 731, 275-284) décrit la purification d'une protéine recombinante de fusion L-sélectine-Ig à partir du surnageant clarifié d'un milieu de culture de CHO par chromatographie sur un support d'affinité comprenant des aptamères.

Liu et al. (Journal of molecular récognition, 2003 ; 16 :23-27) décrit des aptamères d'ARN spécifiques de la thrombine bovine destinés à des applications cliniques.

Zhao et al. (Anal. Chem, 2008, 80, 3915-3920) décrit un procédé de détection du cytochrome C et de la thrombine par chromatographie capillaire sur un support d'affinité d'aptamères.

Bien que les procédés connus de purification des protéines plasmatiques soient satisfaisants, il existe un besoin dans l'état de la technique pour des procédés alternatifs ou améliorés, par rapport aux procédés existants.

## RESUME DE L'INVENTION

L'invention concerne un procédé tel que défini dans les revendications, notamment un procédé pour la purification d'une protéine plasmatique humaine recombinante à partir d'un milieu de départ qui est une solution biologique provenant d'un animal non-humain transgénique pour ladite protéine plasmatique humaine, comprenant les étapes suivantes :

a) mettre en contact un échantillon contenant la protéine plasmatique humaine avec un support d'affinité comprenant un support solide sur lequel sont immobilisés des aptamères désoxyribonucléiques se liant spécifiquement à ladite protéine plasmatique humaine, afin de former des complexes entre (i) les aptamères désoxyribonucléiques immobilisés sur ledit support d'affinité et (ii) ladite protéine plasmatique humaine,
b) libérer la protéine à partir des complexes formés à l'étape a), et
c) récupérer ladite protéine plasmatique humaine sous une forme purifiée.

## DESCRIPTION DES FIGURES

La **figure 1** illustre un profil de chromatographie obtenu lors de la mise en oeuvre du procédé de purification d'un Facteur VII humain recombinant produit dans le lait de lapine avec le support d'affinité dans lequel sont immobilisés des aptamères nucléiques anti-FVII humain. En abscisse ; le temps ; en ordonnées : la valeur d'absorbance (D.O.) à 254 nanomètres.
La **figure 2** représente la courbe des valeurs de mesure de l'absorbance à 280 nm en fonction du temps.
La **figure 3** est un cliché d'un gel d'électrophorèse SDS PAGE avec une coloration au bleu de commassie permettant une quantification relative des bandes. De la gauche vers la droite du gel sur la Figure 3 les pistes représentent les résultats de migration des produits de départ suivants :

- piste « MD » : la composition de départ Betafact®,
- piste « NR » : la fraction non retenue correspondant au pic n° 1 du profil chromatographique de la Figure 2
- piste « E1 » : la fraction d'élution correspondant au pic n°2 du profil chromatographique de la figure 2

La **figure 4** représente la courbe des valeurs de mesure de l'absorbance à 280 nm en fonction du temps.
La **Figure 5** est un cliché d'un gel d'électrophorèse SDS PAGE. De la gauche vers la droite du gel sur la Figure 5 les pistes représentent les résultats de migration des produits de départ suivants :

- piste « E5 » : la composition de départ de lait de truie transgénique contenant du Facteur IX humain transgénique pré-purifié par une étape chromatographique MEP HyperCel,
- piste « E6 » : la fraction non retenue correspondant au pic n° 1 du profil chromatographique de la Figure 4
- piste « E7 » : la fraction d'élution correspondant au pic n°2 du profil chromatographique de la figure 4
- piste « E8 » la fraction de régénération correspond au pic n°3 du profil chromatographique de la figure 4
- piste « T FIX » : Témoin de facteur IX humain plasmatique purifié.

La **Figure 6** représente la courbe des valeurs de mesure de l'absorbance à 280 nm en fonction du temps. Sur la Figure 6, le pic n°1 correspond à la fraction du produit de départ qui n'a pas été retenue sur la colonne. Le pic n°2 correspond à la fraction d'élution.
La **Figure 7** représente un cliché d'un gel du produit de départ ainsi que le produit élué a été analysé en SDS PAGE avec coloration au nitrate d'argent afin de visualiser l'élimination des impuretés : la piste n°1 correspond à la fraction du produit de départ et la piste n°2 à la fraction d'élution. Malgré la pureté importante du produit de départ on constate que la fraction éluée ne contient plus de contaminant ou de formes dégradées
La **Figure 8** représente les courbes de liaison de l'aptamère immobilisé Mapt2 au Facteur VII humain recombinant produit dans le lait d'une lapine transgénique. Les flèches correspondent au temps des différentes injections, respectivement de gauche à droite sur la figure 8 : 1 : injection du Facteur VII recombinant ; 2 : injection d'un tampon à 1M NaCl ; 3 : injection d'un tampon à 2M NaCl, 4 : injection d'un tampon à 3 M NaCl ; 5 : injection d'un tampon Tris 50 mM, EDTA 10 mM. En abscisse: le temps exprimé en secondes; en ordonnées ; la valeur du signal de réponse, exprimé en unités (RU) arbitraires.

La **Figure 9** représente les courbes de liaison de l'aptamère immobilisé Mapt2 au Facteur VII humain recombinant produit dans le lait d'une lapine transgénique. Les flèches correspondent au temps des différentes injections, respectivement de gauche à droite sur la figure 9 : 1 : propylène glycol à 50% ; 2 : EDTA à 10 mM.

## DESCRIPTION DETAILLEE DE L'INVENTION

Après de longues recherches, le demandeur a mis au point un procédé de purification des protéines du plasma sanguin, comprenant une étape de chromatographie au cours de laquelle a lieu une fixation spécifique de la protéine d'intérêt sur un ligand préalablement immobilisé sur le support de chromatographie, puis de libération de la protéine d'intérêt retenue sur ledit support et récupération de la protéine purifiée dans le volume de l'éluat.

Plus précisément, il est fourni selon l'invention un procédé pour la purification des protéines du plasma sanguin comprenant une étape de fixation spécifique de la protéine plasmatique d'intérêt sur un support sur lequel sont immobilisés des aptamères désoxyribonucléiques se liant spécifiquement à ladite protéine d'intérêt, suivie d'une étape de récupération de ladite protéine d'intérêt purifiée.

De manière surprenante, on a montré que des supports de chromatographie d'affinité sur lesquels sont immobilisés des aptamères désoxyribonucléiques spécifiques de la protéine plasmatique d'intérêt, c'est-à-dire y compris sous la forme de composés immobilisés comprenant de tels aptamères désoxyribonucléiques, peuvent être utilisés avec succès dans des procédés d'obtention de protéines plasmatiques, utilisables comme principes actifs d'un médicament.

De manière surprenante, Il est montré que l'on peut fabriquer un support d'affinité tel que défini dans la présente description en utilisant des aptamères d'acide désoxyribonucléique (ADN), pourtant considérés dans l'état de la technique comme des acides nucléiques ligands dont la mise en oeuvre est malaisée, et dont la spécificité pour la protéine cible est moindre que la spécificité de la molécule d'ARN de séquence correspondante. Notamment, il est admis dans l'état de la technique que les ADN ligands ont une flexibilité moindre que l'ARN correspondant et qu'en conséquence sont moins aptes que les ARN ligands à subir des changements de conformation. On rappelle que lorsqu'un aptamère nucléique se lie à la protéine cible, il se produit un changement de conformation. Il a aussi été décrit que plus le changement de conformation de l'aptamère nucléique est rapide et plus l'affinité dudit aptamère nucléique pour la protéine cible est élevée (Michaud et al., 2003, Anal Chem, Vol. 76 : 1015-1020 ; Brumbt et al., 2005, Anal Chem, Vol. 77 : 1993-1998)

On a également montré que le support d'affinité permet de purifier des protéines de la coagulation de séquence primaire très distinctes, telles que le Facteur VII et le Facteur IX humains.

On a aussi montré que le support d'affinité permet de purifier des protéines de la coagulation à partir de milieux de départ de composition complexe, comme une composition plasmatique enrichie en Facteur IX humain et un lait d'animal transgénique pour le Facteur IX humain, avec une grande spécificité de liaison à ladite protéine de la coagulation d'intérêt.

On a également montré que le support d'affinité permet de purifier sélectivement une protéine de la coagulation humaine à partir d'un milieu de départ comprenant également une protéine orthologue non-humaine, par exemple à partir d'un lait d'animal transgénique pour le Facteur IX humain, ledit lait comprenant également du Facteur IX produit naturellement par ledit animal transgénique, ledit Facteur IX produit naturellement par ledit animal transgénique (ou FIX endogène) pouvant être par exemple un FIX porcin.

On a également montré que le support d'affinité permet de purifier sélectivement une protéine de la coagulation humaine à partir d'un milieu de départ comprenant également une protéine orthologue non-humaine, par exemple à partir d'un lait d'animal transgénique pour le Facteur VII humain, ledit lait comprenant également du Facteur VII produit naturellement par ledit animal transgénique, ledit Facteur VII produit naturellement par ledit animal transgénique (ou FVII endogène) pouvant être par exemple un FVII de lapin

Il est décrit un support d'affinité pour la fixation sélective d'une protéine du plasma sanguin, comprenant un matériau support solide sur lequel sont immobilisés des aptamères désoxyribonucléiques se liant spécifiquement à ladite protéine plasmatique, y compris sous la forme de composés comprenant de tels aptamères désoxyribonucléotidiques.

Les molécules d'aptamère désoxyribonucléique se liant spécifiquement à la protéine plasmatique d'intérêt, ainsi que les composés comprenant de tels aptamères désoxyribonucléiques, constituent des sites de liaison spécifique de ladite protéine cible portés par ledit matériau support solide.

Par « aptamères désoxyribonucléiques », on englobe des acides désoxyribonucléiques monobrin ayant une longueur de 5 à 120 nucléotides de longueur et qui se lient spécifiquement à une protéine du plasma sanguin. Par « composés » comprenant un aptamère désoxyribonucléique, on englobe également des composés qui comprennent dans leur structure lesdits acides désoxyribonucléiques définis précédemment. Ainsi, les composés comprenant un acide désoxyribonucléique se liant spécifiquement à une protéine plasmatique, humaine ou de mammifère, englobent les composés dans lesquels ledit acide désoxyribonucléique est inclus dans une structure comprenant une molécule de biotine.

On a montré qu'un support d'affinité tel que défini ci-dessus permettait une immobilisation efficace de la protéine plasmatique d'intérêt, qui peut aussi être appelée « protéine cible » dans la présente description.

Un support d'affinité tel que défini ci-dessus possède une haute capacité d'adsorption de la protéine cible, puisque la mise en contact du support solide avec une solution liquide contenant la protéine plasmatique à purifier permet de saturer au moins 50 pour cent des sites cibles portés par le support solide.

On a également montré que les molécules de la protéine plasmatique qui sont fixées spécifiquement sur les molécules d'aptamères désoxyribonucléiques, peuvent ensuite être éluées du support d'affinité avec un bon rendement, d'au moins 75 pour cent.

De plus, on a montré que le support d'affinité possède une grande spécificité pour la protéine plasmatique d'intérêt, puisque ladite protéine plasmatique peut être retrouvée avec un degré de pureté allant jusqu'à 99,95 pour cent en poids, par rapport au poids total des protéines contenues dans l'éluat.

Comme cela est illustré dans les exemples, un accroissement de deux ordres de grandeur de la pureté de la protéine de la coagulation d'intérêt peut encore être obtenu avec un support d'affinité tel que décrit ici, en utilisant comme produit de départ une composition comprenant la protéine cible de la coagulation à un haut degré de pureté, par exemple à plus de 98% en poids, par rapport au poids total des protéines contenues dans ledit produit de départ. On précise qu'un tel accroissement de pureté est obtenu y compris lorsque les impuretés présentes dans le produit de départ possèdent des caractéristiques structurelles ou physico-chimiques très proches de celles de la protéine cible de la coagulation que l'on cherche à purifier.

De manière importante, on a montré que les caractéristiques ci-dessus de haute capacité d'absorption, de bon rendement et de haute spécificité du support d'affinité étaient obtenues notamment pour la purification de la protéine plasmatique d'intérêt à partir d'un milieu de départ de composition complexe, tel que du plasma sanguin ou encore un fluide biologique d'un mammifère transgénique pour ladite protéine plasmatique d'intérêt.

On a également montré que l'immobilisation des aptamères nucléiques sur le matériau support solide était irréversible et durable, puisque la présence d'aptamères désoxyribonucléiques détachés du support n'est pas détectable dans la solution d'éluat.

Notamment, on a montré que le support d'affinité peut être « régénéré », par élimination des protéines restées fixées sur le support après élution, de très nombreuses fois sans altération significative de (i) sa capacité d'absorption de la protéine plasmatique cible, (ii) sa spécificité vis-à-vis de ladite protéine cible, ou encore (iii) son absence de relarguage des aptamères désoxyribonucléiques immobilisés sur le matériau support solide. De plus, la régénération du support d'affinité peut être réalisée selon des techniques connues et avec des agents de régénération connus, telle que l'urée.

Les acides désoxyribonucléiques utilisés comme ligands de la protéine plasmatique d'intérêt présentent de nombreux avantages. De par leur nature oligonucléotidique les aptamères possèdent une faible immunogénicité et une résistance importante à des conditions physico-chimiques stringentes (présence d'urée, de DMSO, d'un pH très acides ou très basique, utilisation de solvants organiques ou de température élevée) permettant des stratégies variées de contrôle de la sécurité sanitaire, en particulier de la sécurité vis-à-vis des virus ou d'agents pathogènes non conventionnels, dans le cadre d'un usage en tant que ligand d'affinité. De plus leur sélectivité est importante. Enfin, comme déjà mentionné, la production d'aptamères désoxyribonucléiques implique des coûts relativement limités.

Ainsi, la combinaison de caractéristiques ci-dessus du support d'affinité matérialisent l'aptitude dudit support d'affinité à être utilisé comme moyen pour la purification d'une protéine du plasma sanguin, puisque

- ledit support d'affinité permet la mise en oeuvre de procédés de purification d'une protéine plasmatique ayant un très haut degré de pureté,
- ledit support d'affinité ne libère pas de manière détectable de substances indésirables, en particulier de molécules d'aptamères désoxyribonucléiques, dans la solution d'éluat contenant la protéine plasmatique purifiée,
- l'éventuel détachement de molécules d'aptamères désoxyribonucléiques n'entraîne pas d'inconvénients pour la santé humaine,
- la fixation, puis l'élution de la protéine plasmatique d'intérêt sur le support d'affinité, lorsqu'il s'agit d'une protéine plasmatique à activité enzymatique, n'entraîne pas la formation de quantités détectables de la forme activée de ladite protéine plasmatique,
- ledit support d'affinité est peu onéreux à fabriquer, notamment en raison des coûts réduits de la production des aptamères désoxyribonucléiques, et
- la mise en oeuvre dudit support d'affinité pour la purification d'une protéine plasmatique est elle-même peu onéreuse du fait de la longévité dudit support, due en particulier à la possibilité de le régénérer de nombreuses fois, et sur une longue période de temps.

De plus, comme cela a été déjà mentionné, le support d'affinité est apte à fixer sélectivement, de manière réversible, la protéine plasmatique cible, avec un bon rendement et une bonne spécificité, dans des procédés de purification à partir de milieux de constitution complexe, en particulier à partir de milieux classiquement utilisés à l'échelle industrielle, tels que le plasma humain ou des milieux de culture ou des fluides biologiques contenant ladite protéine d'intérêt.

De plus, comme cela sera détaillé plus loin dans la présente description, le support d'affinité est apte au traitement de grands volumes de solution contenant la protéine plasmatique d'intérêt. Le support d'affinité constitue donc un outil de purification d'une protéine du plasma sanguin qui est parfaitement bien adapté à une utilisation à l'échelle industrielle.

D'autres avantages du support d'affinité seront spécifiés ultérieurement dans la présente description, soit en relation avec la description du support d'affinité lui-même, soit en relation avec les procédés de purification d'un protéine plasmatique dans lesquels ledit support d'affinité peut être utilisé.

A la connaissance du demandeur, Il est décrit ici pour la première fois un support d'affinité comprenant des aptamères désoxyribonucléiques immobilisés pour la purification d'une protéine du plasma sanguin dans des conditions d'utilisation à l'échelle industrielle. On connait par ailleurs dans l'état de la technique une variété d'aptamères nucléiques spécifiques de la thrombine, du Facteur VII et du Facteur IX (voir demande PCT n° WO 02/26932). Toutefois, à la connaissance du demandeur, l'utilisation d'aptamères désoxyribonucléiques pour la fabrication de supports d'affinité utilisables à l'échelle industrielle dans des procédés de purification de protéines plasmatiques n'a jamais été décrit dans l'état de la technique.

Par « fixation sélective », on entend aux fins de la présente description la liaison spécifique de manière non covalente de la protéine plasmatique d'intérêt aux acides désoxyribonucléiques immobilisés constitutifs du support d'affinité, et qui est réversible par mise en contact du support d'affinité sur lequel se sont formés des complexes non covalents entre lesdits acides désoxyribonucléiques et ladite protéine d'intérêt avec une solution de constitution adaptée, qui peut être appelée aussi solution d'élution.

Par « protéine plasmatique », on entend toute protéine, spécialement toute protéine d'intérêt industriel ou thérapeutique, contenue dans le plasma sanguin. Les protéines du plasma sanguin englobent l'albumine, alpha/macroglobuline, antichymotrypsine, antithrombine, antitrypsine, Apo A, Apo B, Apo C, Apo D, Apo E, Apo F, Apo G, beta XIIa, C 1-inhibiteur, protéine C-réactive, C7, C1r, C1s, C2 C3, C4, C4bP, C5, C6, C1q, C8, C9, carboxypeptidase N, céruloplasmine, Facteur B, Facteur D, Facteur H, Facteur I, Facteur IX, Facteur V, Facteur VII, Facteur VIIa, Facteur VIII, Facteur X, Facteur XI, Facteur XII, Facteur XIII, Fibrinogène, Fibronectine, Haptoglobine, Hemopexine, Héparine cofacteur II, Histidine rich GP, IgA, IgD, IgE, IgG, ITI, IgM, Kininase II, Kininogène HPM, Lysozyme, PAI 2, PAI I, PCI, Plasmine, Inhibiteur de la plasmine, Plasminogène, Préalbumine, Prokallicréine, Properdine, Protéase Nexine INH, Protéine C, Protéine S, Protéine Z, Prothrombine, TFPI, Thiol-protéinase, Thrombomoduline, Facteur tissulaire (TF), TPA, Transcolabamine II, Transcortine, Transferrine, Vitronectine, et le Facteur de Willebrand.

Notamment, les protéines plasmatiques englobent les protéines de la coagulation, c'est-à-dire les protéines plasmatiques impliquées dans la chaîne de réactions en cascade aboutissant à la formation d'un caillot sanguin. Les protéines de la coagulation englobent le Facteur I (fibrinogène), le Facteur II (prothrombine), le Facteur V (proaccélérine), le Facteur VII (proconvertine), le Facteur VIII (facteur anti-hémophilique A), le Facteur IX (facteur anti-hémophilique B), le Facteur X (Facteur Stuart), le Facteur XI (Facteur Rosenthal ou PTA), le Facteur XII (Facteur Hageman), le Facteur XIII (Facteur stabilisant la fibrine ou FSF), la PK (Prékalicrine) le KHPM (kininogène de haut poids moléculaire), la thromboplastine tissulaire, le cofacteur II de l'héparine (HCII), la protéine C (PC), la thrombomoduline (TM), la protéine S (PS), le facteur de Willebrand (Wf) et l'inhibiteur de la voie du facteur tissulaire (TFPI), ou encore les facteurs tissulaires.

Dans certains modes de réalisation, la protéine plasmatique consiste en une protéine de la coagulation à activité enzymatique.

Les protéines de la coagulation à activité enzymatique englobent le Facteur II (prothrombine), le Facteur VII (proconvertine), le Facteur IX (facteur anti-hémophilique B), le Facteur X (Facteur Stuart), le Facteur XI (Facteur Rosenthal ou PTA), le Facteur XII (Facteur Hageman), le Facteur XIII (Facteur stabilisant la fibrine ou FSF) et la PK (Prékalicrine).

Selon l'invention, la protéine plasmatique consiste en une protéine plasmatique humaine, recombinante.

Dans des modes de réalisation préférés, la protéine plasmatique est le Facteur VII humain, recombinant.

De manière générale, les supports solides sur lesquels peuvent être immobilisés les aptamères englobent tout type de support ayant la structure et la composition retrouvée communément pour les supports de filtre, des membranes, etc. Les supports solides englobent notamment les résines, les résines pour colonne de chromatographie d'affinité, les billes de polymères, les billes magnétiques, les billes paramagnétiques, les matériaux supports de membrane filtrante, etc. Les supports solides englobent aussi notamment les matériaux à base de verre ou de métal, tels que l'acier, l'or, l'argent, l'aluminium, le cuivre, le silicium, le verre, la céramique. Les supports solides englobent aussi notamment des matériaux polymères, tels qu'un polyéthylène, un polypropylène, un polyamide, un fluorure de polyvinylidène, et des combinaisons de ceux-ci.

Dans certains modes de réalisation, le support solide peut être revêtu avec un matériau facilitant l'attachement, la fixation, la formation de complexes, l'immobilisation ou l'interaction avec les aptamères, ou avec les composés comprenant lesdits aptamères..

Dans certains modes de réalisation, le support solide est une lame de verre dont la surface est revêtue d'une couche d'or, d'une couche ayant subi un traitement par carboxyméthylation, d'une couche de dextran, de collagène, d'avidine, de streptavidine, etc.

De cette manière, les aptamères, ou les composés comprenant lesdits aptamères , peuvent être immobilisés sur le support solide par l'intermédiaire d'un revêtement d'attachement, comme par exemple décrit ci-dessus, soit par réaction chimique avec création de liaisons covalentes, soit par association par des liaisons non covalentes telles que des liaisons hydrogène, des forces électrostatiques, des forces de Van der Waals, etc.

Les exemples décrivent des modes de réalisation d'un support d'affinité dans lesquels les aptamères désoxyribonucléiques sont immobilisés, par l'intermédiaire des composés dans la structure desquels ils sont inclus, par des liaisons non covalentes au matériau de support solide.

Dans les exemples, on décrit notamment des supports d'affinité comprenant un matériau de support solide à la surface duquel sont greffées des molécules de streptavidine, les aptamères désoxyribonucléiques étant inclus dans la structure de composés comprenant des aptamères couplés, à une de leurs extrémités, à une molécule de biotine, et lesdits aptamères étant immobilisés sur ledit matériau de support solide par immobilisation non covalente entre les molécules de streptavidine du matériau support et les molécules de biotine des composés comprenant lesdits aptamères désoxyribonucléiques.

Dans les exemples, on décrit un mode de réalisation d'un support d'affinité comprenant un matériau support sur lequel sont greffées des molécules de streptavidine. De tels matériaux support solide sont facilement disponibles dans le commerce.

Par « acides désoxyribonucléiques se liant spécifiquement à une protéine plasmatique », ou « aptamères » ou « aptamères nucléiques », on entend des molécules d'ADN (acide désoxyribonucléique) possédant la capacité à se lier à une protéine plasmatique d'intérêt donnée, supérieure à la capacité à se lier à toute autre protéine.

Dans certains modes de réalisation, les aptamères nucléiques constitutifs du support d'affinité ont la capacité à se lier à une protéine plasmatique humaine donnée, supérieure à la capacité à se lier à toute autre protéine humaine.

Dans certains modes de réalisation, les aptamères nucléiques ont notamment la capacité à se lier à une protéine plasmatique humaine donnée, supérieure à la capacité à se lier à toute autre protéine plasmatique homologue codée par le génome d'un mammifère non humain. De manière illustrative, un aptamère nucléique spécifique du Facteur VII humain, utilisable dans un support d'affinité, possède une capacité à se lier au Facteur VII humain supérieure à la capacité à se lier au Facteur VII provenant d'un mammifère non humain, y compris au Facteur VII de lapin.

Au sens de la présente description, un premier aptamère désoxyribonucléique nucléique possède une capacité à se lier au Facteur VII/VIIa humain, supérieure à celle d'un second aptamère désoxyribonucléique de masse équivalente, lorsque, en utilisant l'une quelconque des techniques de détection de liaison ci-dessus et dans les mêmes conditions d'essai, une valeur de signal de liaison supérieure statistiquement significative est obtenue avec le premier aptamère désoxyribonucléique, par rapport à celle obtenue avec les second aptamère désoxyribonucléique. De manière illustrative, lorsque la technique de détection de liaison utilisée est la technique Biacore®, un premier aptamère désoxyribonucléique possède une capacité à se lier au Facteur VII/VIIa humain, supérieure à celle d'un second aptamère désoxyribonucléique de masse équivalente, lorsque la valeur de signal de résonance pour le premier aptamère désoxyribonucléique, quelle que soit l'unité de mesure de résonance exprimée, est statistiquement supérieure à la valeur de signal de résonance mesurée pour le second aptamère désoxyribonucléique. Deux valeurs de mesure « statistiquement » distinctes englobent deux valeurs qui on entre elles un écart supérieur à l'erreur de mesure de la technique de détection de liaison utilisée.

Préférentiellement, un aptamère désoxyribonucléique du support d'affinité possède une forte affinité pour la protéine plasmatique cible. Préférentiellement, ledit aptamère désoxyribonucléique possède une valeur de Kd, vis-à-vis de ladite protéine plasmatique cible, inférieure à 500 nM. La valeur de Kd peut être mesurée selon la technique Biacore®.

A titre illustratif, on a montré que l'aptamère nucléique comprenant la séquence SEQ ID N° 86 a une capacité à se lier au Facteur VII/VIIa humain qui est significativement supérieure à sa capacité de liaison à n'importe quel Facteur VII/VIIa provenant d'un mammifère non humain. En particulier, alors qu'un aptamère comprenant l'acide nucléique de séquence SEQ ID N° 86 possède une forte capacité à se lier à tout type de Facteur VII/VIIa humain, y compris un Facteur VII/VIIa naturel ou un Facteur VII/VIIa recombinant, il a une capacité faible ou nulle à se lier à un Facteur VII/VIIa codé par le génome d'un mammifère non humain, y compris un Facteur VII/VIIa de lapin.

Plus précisément, pour l'aptamère comprenant l'acide nucléique de séquence SEQ ID N° 86, on a déterminé selon la technique Biacore®, que la valeur de capacité de liaison au Facteur VII/VIIa humain, exprimée comme la constante de dissociation Kd, est d'environ 100 nM. De plus, ledit aptamère nucléique possède une capacité de liaison identique à la fois au Facteur VII/VIIa humain plasmatique et à un Facteur VII/VIIa humain recombinant, par exemple produit chez un lapin transgénique

On a aussi montré que les complexes entre un aptamère comprenant l'acide nucléique de séquence SEQ ID N° 86 et un Facteur VII/VIIa humain sont stoechiométriques, c'est à dire que le rapport du nombre de molécules d'acide

nucléique de séquence SEQ ID N°86 au nombre de molécules de Facteur VII/VIIa humain complexées est d'environ 1::1, et peut être en particulier de 1::1.

Selon l'invention, comme cela a déjà été mentionné précédemment, le support d'affinité peut être utilisé dans une étape d'un procédé de purification d'une protéine plasmatique humaine recombinante produite par un mammifère transgénique non humain. Dans de tels modes de réalisation d'un procédé de purification d'une protéine plasmatique, le milieu complexe de départ comprend la protéine recombinante humaine en mélange avec de nombreuses protéines produites naturellement par ledit mammifère transgénique, y compris, le cas échéant, la protéine plasmatique homologue à la protéine recombinante humaine. On comprend que, dans de tels modes de réalisation, il est avantageux que les aptamères nucléiques constitutifs du support d'affinité se fixent sélectivement sur la protéine humaine d'intérêt, et ne se fixe pas, dans les mêmes conditions opératoires, sur la protéine homologue produite naturellement par le mammifère non humain.

Selon un aspect particulier des aptamères nucléiques constitutifs du support d'affinité, la capacité desdits aptamères à se lier « spécifiquement » à la protéine plasmatique humaine d'intérêt peut aussi être exprimé comme le rapport des constantes de dissociation Kd, respectivement pour la protéine humaine et pour la protéine homologue de mammifère non humain.

Selon encore une autre caractéristique d'un aptamère nucléique constitutif du support d'affinité, la capacité dudit aptamère nucléique à se lier spécifiquement à la protéine plasmatique humaine peut aussi être exprimée par la condition (A) suivante :

$$\text{Kd humain/Kd non-humain} < 0{,}01 \quad (A),$$

dans laquelle :

- « Kd humain » est la constante de dissociation d'un aptamère nucléique ladite protéine humaine, exprimée en unités molaires, et
- « Kd non-humain » est la constante de dissociation dudit aptamère nucléique pour la protéine homologue non humaine, exprimée dans les mêmes unités molaires.

Préférentiellement, pour une mise en oeuvre optimale du support d'affinité dans des procédés de purification d'une protéine plasmatique humaine recombinante, un aptamère nucléique se liant spécifiquement à ladite protéine humaine recombinante peut être défini aussi par un rapport Kd humain/Kd non-humain inférieur à 0,005.

Les aptamères nucléiques constitutifs du support d'affinité peuvent être préparés selon la technique appelée SELEX. Le terme « aptamère » tel qu'utilisé englobe une molécule d'acide désoxyribonucléique (ADN) monobrin capable de se lier de manière spécifique à une protéine. Les aptamères comprennent généralement entre 5 et 120 nucléotides et peuvent être sélectionnés *in vitro* selon un procédé connu sous le nom de SELEX (Systematic Evolution of Ligands by Exponential Enrichment), qui a été initialement décrit notamment dans la demande PCT N° WO 1991/019813). Le procédé SELEX de sélection des aptamères consiste, en ce qui concerne l'obtention des aptamères ADN utilisés tels que décrits ici, à mettre en présence une protéine avec une librairie combinatoire d'acides désoxyribonucléiques (en général 1015 molécules), les acides désoxyribonucléiques ne se liant pas à la cible sont éliminés, les acides désoxyribonucléiques se liant à la cible sont isolés et amplifiés. Le procédé est répété jusqu'à ce que la solution soit suffisamment enrichie avec les acides désoxyribonucléiques ayant une bonne affinité pour la protéine d'intérêt (Tuerk et Gold, « Systematic evolution of ligands by exponential enrichment : RNA ligands to bacteriophage T4 DNA polymerase » (1990) Science, 249(4968) :505-10 et Ellington et Szostak, « In vitro selection of RNA molecules that bind specific ligands », (1990) Nature Aug 30;346(6287):818-22). D'autres exemples de procédé SELEX sont donnés dans les documents EP 0 786 469, EP 668 931, EP 1 695 978, EP 1 493 825 dont les enseignements peuvent être repris dans la réalisation du procédé de sélection d'un aptamère désoxyribonucléique utilisé selon le procédé de purification de l'invention.

Pour son utilisation dans un moyen de purification du Facteur VII/VIIa humain, un acide désoxyribonucléique se liant spécifiquement à une protéine plasmatique d'intérêt est préférentiellement inclus dans une structure chimique, aussi appelée « composé » dans la présente description, comprenant également un moyen espaceur et, le cas échéant, un moyen d'immobilisation sur un support solide.

Dans certains modes de réalisation, l'aptamère désoxyribonucléique est inclus dans la structure d'un composé de formule (I) suivante :

$$[FIX]_x\text{-}[SPAC]_y\text{-}[APT] \qquad (I)$$

, dans laquelle :

- [FIX] signifie un composé d'immobilisation sur un support,
- [SPAC] signifie une chaîne espaceur,
- [APT] signifie un acide désoxyribonucléique se liant spécifiquement à une protéine plasmatique, aussi désigné aptamère désoxyribonucléique,
- x est un entier égal à 0 ou 1, et
- y est un entier égal à 0 ou 1.

La « chaîne espaceur » désignée [SPAC] dans le composé de formule (I) peut être de tout type connu. Ladite chaîne espaceur a pour fonction d'éloigner physiquement l'acide désoxyribonucléique de la surface du support solide sur lequel ledit composé peut être immobilisé et de permettre une mobilité relative de l'acide nucléique par rapport à la surface du support solide sur lequel il peut être immobilisé. La chaîne espaceur limite ou évite que des encombrements stériques, dus à une trop grande proximité du support solide et de l'acide nucléique, gênent les évènements de liaison entre ledit acide nucléique et des molécules de protéine plasmatiques susceptibles d'être mises en contact avec celui-ci.

Dans le composé de formule (I), la chaîne espaceur est liée préférentiellement à l'extrémité 5' ou à l'extrémité 3' de l'acide désoxyribonucléique aptamère.

Avantageusement la chaîne espaceur est liée à la fois à une extrémité de l'aptamère et au support solide. Cette construction avec espaceur a pour avantage de ne pas immobiliser directement l'aptamère sur le support solide. De préférence la chaîne espaceur est un oligonucléotide non spécifique ou du Polyéthylène Glycol (PEG). Lorsque la chaîne espaceur consiste en un oligonucléotide non spécifique, ledit oligonucléotide comprend avantageusement au moins 5 nucléotides de longueur, de préférence entre 5 et 15 nucléotides de longueur.

Selon certains aspects d'un composé de formule (I) dans lesquels la chaîne espaceur consiste en un Polyéthylène Glycol, ladite chaîne espaceur englobe un polyéthylène glycol de type PEG(C18), commercialisé par exemple par la Société Sigma Aldrich

Pour immobiliser l'aptamère sur la chaîne espaceur, l'acide désoxyribonucléique peut être modifié chimiquement avec différents groupements chimiques tels que des groupements permettant d'immobiliser ledit acide nucléique de façon covalente, tels que des thiols, des amines ou tout autre groupement capable de réagir avec des groupements chimiques présents sur le support solide.

Dans le composé de formule (I) le composé [FIX] consiste en un composé choisi parmi (i) un composé capable de former une ou plusieurs liaisons covalente(s) avec le matériau de support solide et (ii) un composé capable de se lier spécifiquement sur le support solide par l'intermédiaire de liaison faibles non covalentes, y compris des liaisons hydrogènes, des forces électrostatiques ou des forces de Van der Waals.

Le premier type de composé [FIX] englobe les agents de couplage bifonctionnels, comme le glutaraldéhyde, le SIAB ou encore le SMCC.

Le composé SIAB, décrit par Hermanson G.T. (1996, Bioconjugate techniques, San Diego : Academic Press, pp 239-242), est le composé de formule (I) suivante :

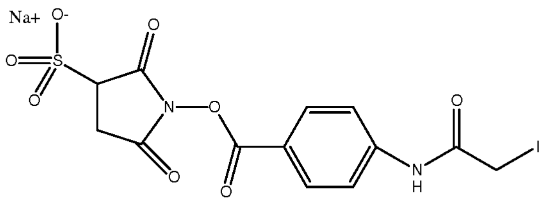

(I)

Le composé SIAB comprend deux groupes réactifs, respectivement un groupe iodoacétate et un groupe ester Sulfo-NHS, ces groupes réagissant respectivement sur des groupes amino et sulfhydryl.

Le composé SMCC, qui est décrit par Samoszuk M.K. et al. (1989, Antibody, Immunoconjugates Radiopharm., 2(1) : 37-46), est le composé de formule (II) suivante :

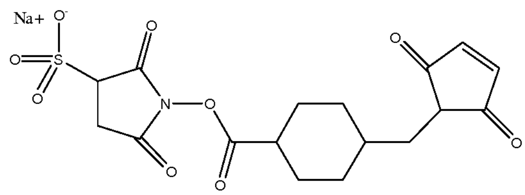

(II)

Le composé SMCC comprend deux groupes réactifs, respectivement un groupe ester Sulfo-NHS et un groupe maléimide, qui réagissent respectivement avec un groupe amino et un groupe sulfhydryl.

Le second type de composé [FIX] englobe la biotine, qui est capable de se lier de manière spécifique non covalente à des molécules d'avidine ou de streptavidine présentes sur le support mobile.

On connaît déjà dans l'état de la technique des aptamères nucléiques capables de se lier à diverses protéines impliquées dans la voie de la coagulation sanguine, y compris des aptamères liant le Facteur de Willebrand (Demande PCT n° WO 2008/150495), des aptamères liant l'alpha-thrombine (demande de brevet euopéen n° EP 1 972 693) ou la thrombine (Zhao et al., 2008, Anal Chem, Vol. 80(19) : 7586-7593), des aptamères liant le Facteur IX/IXa (Subash et al., 2006, Thromb Haemost, Vol. 95 : 767-771; Howard et al., 2007, Atherioscl Thromb Vasc Biol, Vol. 27 : 722-727; Demande PCT n° WO 2002/096926; Brevet aux Etats-Unis n° US 7,312,325), des aptamères liant le Facteur X/Xa (Demande PCT n° WO 2002/096926; Brevet aux Etats-Unis n° US 7,312,325).

Des aptamères nucléiques se liant au facteur VII/VIIa humain ont aussi été décrits dans l'état de la technique (Rusconi et al., 2000, Thromb Haemost, Vol. 84(5) : 841-848; Layzer et al., 2007, Spring, Vol. 17 : 1-11).

On précise qu'aucun des aptamères ci-dessus, qui consistent principalement, sinon exclusivement, en des aptamères ribonucléotidiques, n'est décrit pour son utilisation pour purifier la protéine cible sur laquelle ils se lient.

Comme déjà mentionné, un avantage spécifique des aptamères désoxyribonucléiques concerne leur facilité de fabrication, comparée aux difficultés de synthèse des aptamères ARN, ainsi que leur prix de revient qui est significativement plus réduit que le prix de revient d'un aptamère ARN.

La présente description décrit également un dispositif de chromatographie d'affinité pour la purification d'une protéine plasmatique, comprenant un conteneur dans lequel est disposé une quantité adaptée d'un support d'affinité tel que défini dans la présente description.

Des formes variées de conteneurs pour supports de chromatographie sont connus dans l'état de la technique et sont englobés par la signification du terme « conteneur » ci-dessus. Les caractéristiques importantes d'un tel conteneur englobent la présence d'un moyen d'alimentation du dispositif de chromatographie d'affinité en échantillon de départ et d'un moyen de sortie du liquide après sa mise en contact avec le support d'affinité.

Il est décrit aussi un procédé pour immobiliser une protéine plasmatique sur un support, comprenant une étape au cours de laquelle on met en contact un échantillon contenant ladite protéine plasmatique d'intérêt avec un support d'affinité tel que défini ci-dessus.

Par « échantillon contenant une protéine plasmatique », on entend en général tout type de solution liquide dans laquelle ladite protéine plasmatique est en suspension ou est solubilisée. Des modes de réalisation spécifiques d'un tel échantillon, en particulier en relation avec le procédé de purification décrit ci-après, seront définis ultérieurement dans la présente description.

La présente invention a pour objet un procédé pour la purification d'une protéine plasmatique comprenant les étapes suivantes :

a) mettre en contact un échantillon contenant une protéine plasmatique avec un support d'affinité tel que défini dans la présente description, afin de former des complexes entre (i) les aptamères désoxyribonucléiques immobilisés sur ledit support d'affinité et (ii) ladite protéine plasmatique, et

b) libérer la protéine à partir des complexes formés à l'étape a), et

c) récupérer ladite protéine plasmatique sous une forme purifiée.

Selon l'invention, la protéine plasmatique est une protéine plasmatique humaine recombinante. L'échantillon contenant la protéine plasmatique d'intérêt consiste en un échantillon liquide qui contient ladite protéine d'intérêt, y compris un échantillon liquide comprenant ladite protéine d'intérêt et qui est susceptible de contenir aussi des molécules de la protéine plasmatique homologue d'un mammifère non humain. Selon l'invention, ledit échantillon consiste en une solution biologique, tels qu'un fluide corporel, une cellule, un broyat cellulaire, un tissu, un broyat tissulaire, un organe ou un organisme entier.

Dans certains modes de réalisation du procédé de purification ci-dessus, ledit échantillon consiste en une solution biologique liquide provenant d'un animal tel que du sang, un dérivé du sang (dérivé sanguin), du lait de mammifère ou un dérivé du lait de mammifère. Ledit échantillon peut consister en du plasma, du cryoprécipité du plasma, du lait clarifié ou leurs dérivés.

Selon l'invention, ledit échantillon provient d'un animal transgénique pour la protéine d'intérêt humaine. Avantageusement la solution est du lait de mammifère ou un dérivé du lait de mammifère transgénique pour ladite protéine d'intérêt humaine. Au sens de l'invention, les animaux transgéniques englobent (i) les mammifères non humains tels que les vaches, les chèvres, les lapins, les porcs, les singes, les rats ou les souris, (ii) les oiseaux ou encore (iii) les insectes tels que les moustiques, les mouches ou les vers à soie. Dans certains modes de réalisation préférés, l'animal transgénique pour la protéine d'intérêt humaine est un mammifère transgénique non humain, de manière tout à fait préférée une lapine transgénique pour ladite protéine d'intérêt humaine. Avantageusement, le mammifère transgénique produit ladite protéine d'intérêt humaine recombinante dans ses glandes mammaires, du fait de l'insertion dans son génome d'une cassette d'expression comprenant acide nucléique codant ladite protéine d'intérêt qui est placé sous le contrôle d'un promoteur spécifique permettant l'expression de la protéine transgénique dans le lait dudit mammifère transgénique.

Une méthode de production de ladite protéine plasmatique humaine dans le lait d'un animal transgénique peut comprendre les étapes suivantes : une molécule d'ADN comprenant un gène codant la protéine d'intérêt, ledit gène étant sous le contrôle d'un promoteur d'une protéine sécrétée naturellement dans le lait (tels que le promoteur de la caséine, de la béta-caséine, de la lactalbumine, de la béta-lactoglobuline ou le promoteur WAP) est intégrée dans un embryon d'un mammifère non humain. L'embryon est ensuite placé dans une femelle de mammifère de la même espèce. Une fois que le mammifère issu de l'embryon s'est suffisamment développé, la lactation du mammifère est induite, puis le lait collecté. Le lait contient alors la protéine humaine recombinante d'intérêt.

Un exemple de procédé de préparation de protéine dans le lait d'une femelle de mammifère autre que l'être humain est donné dans le document EP 0 527 063, dont l'enseignement peut être repris pour la production de la protéine d'intérêt selon l'invention. Un plasmide contenant le promoteur WAP (Whey Acidic Protein) est fabriqué par introduction d'une séquence comportant le promoteur du gène WAP, ce plasmide étant réalisé de manière à pouvoir recevoir un gène étranger placé sous la dépendance du promoteur WAP. Le plasmide contenant le promoteur et le gène codant pour la protéine de l'invention sont utilisés pour obtenir des lapines transgéniques, par micro-injection dans le pronucléus mâle d'embryons de lapines. Les embryons sont ensuite transférés dans l'oviducte de femelles hormonalement préparées. La présence des transgènes est révélée par la technique de Southern à partir de l'ADN extrait des lapereaux transgéniques obtenus. Les concentrations dans le lait des animaux sont évaluées à l'aide de tests radio-immunologiques spécifiques.

D'autres documents décrivent des procédés de préparation de protéines dans le lait d'une femelle de mammifère autre que l'homme. On peut citer, sans s'y limiter les documents US 7,045,676 (souris transgénique) et EP 1 739 170. (production de facteur von Willebrand dans un mammifère transgénique).

Il est également décrit que le procédé de purification est également parfaitement adapté à l'obtention d'une protéine plasmatique purifiée à partir d'un échantillon de plasma sanguin humain, ou d'une fraction de plasma sanguin humain, par exemple la fraction cryoprécipitée de plasma sanguin humain.

Selon l'invention, la protéine du plasma sanguin cible est humaine.

Dans certains modes de réalisation du procédé de purification selon l'invention, l'échantillon comprend au moins une protéine du plasma sanguin non-humaine.

Dans certains modes de réalisation du procédé de purification selon l'invention, ladite protéine du plasma sanguin humaine est homologue à ladite protéine du plasma non-humaine.

Dans certains modes de réalisation du procédé de purification selon l'invention, ladite protéine du plasma sanguin humaine est l'homologue de ladite protéine du plasma non-humaine.

Il est décrit que l'échantillon de départ peut consister en le matériel brut, soit l'échantillon de plasma sanguin humain, ou une fraction de celui-ci. Dans certains modes de réalisation du procédé de purification selon l'invention, l'échantillon de départ peut consister en un fluide corporel d'un mammifère non humain transgénique pour la protéine d'intérêt, et qui contient ladite protéine d'intérêt à purifier. Les fluides corporels d'un mammifère non humain transgénique englobent le lait ou une fraction du lait, par exemple une fraction délipidée du lait ou bien encore une fraction appauvrie en micelles de caséine.

Toutefois, le mode de réalisation ci-dessus n'est pas le mode de réalisation privilégié du procédé de purification de l'invention, notamment en raison du risque de colmatage du support d'affinité par les nombreuses protéines présentes dans l'échantillon brut de départ.

Dans des modes de réalisation préférés, ledit échantillon de départ consiste en une solution liquide contenant la protéine plasmatique d'intérêt en suspension dans ladite solution, ladite solution liquide consistant en un produit intermédiaire généré au cours d'un procédé de purification multi-étapes d'une protéine plasmatique.

A titre illustratif, pour un procédé de purification d'une protéine plasmatique selon l'invention à partir d'un fluide

corporel d'un mammifère non humain transgénique pour ladite protéine, l'échantillon de départ peut consister en un éluat d'une chromatographie d'échange d'ions pratiquée à partir d'un filtrat de lait dudit mammifère transgénique non humain. Ce mode particulier de réalisation d'un procédé de purification selon l'invention est illustré dans les exemples. Il est décrit que pour un procédé de purification d'une protéine plasmatique d'intérêt à partir de plasma humain, l'échantillon de départ peut consister en un filtrat d'une étape de filtration en profondeur pratiquée sur la fraction cryoprécipitée d'un échantillon de plasma humain.

De manière générale, les conditions d'utilisation du support d'affinité pour réaliser le procédé de purification de l'invention sont très similaires aux conditions d'utilisation conventionnelles d'un support de chromatographie classique, par exemple du type support d'immuno-affinité sur lequel sont immobilisé des anticorps ligands. L'homme du métier peut par exemple se référer à l'ouvrage de Bailon et al. (Pascal Bailon, George K. Ehrlich, Wen-Jian Fung and Wolfgang Berthold, An Overview of Affinity Chromatography, Humana Press, 2000).

Toutefois, comme cela sera détaillé dans la suite de la description, les conditions de l'étape c) d'élution du procédé de l'invention sont très avantageuses, pour la purification d'une protéine plasmatique.

A l'étape a), un volume approprié de l'échantillon à purifier est mis en contact avec le support d'affinité. Des complexes sont formés entre (i) les aptamères nucléiques immobilisés sur ledit support d'affinité et (ii) la protéine plasmatique d'intérêt contenue dans l'échantillon à purifier.

On a montré dans les exemples que les conditions de capture du Facteur VII sont améliorées quand on utilise à l'étape a) un tampon à concentration réduite en MgCl2 ou même un tampon exempt de MgCl2.

Par tampon à concentration réduite en MgCl2, on entend selon l'invention un tampon dont la concentration finale en MgCl2 est inférieure à 1mM.

Un tampon dont la concentration en MgCl2 est inférieure à 1 mM englobe les tampons dont la concentration en MgCl2 est inférieure à 0,5 mM, 0,1 mM, 0,05 mM et 0,01 mM, avantageusement égale à 0 mM.

Dans un mode de réalisation particulier, le procédé comprend une étape a') laver le support d'affinité avec un tampon de lavage. Avantageusement, le procédé comprend une étape a') laver le support d'affinité en augmentant la force ionique, c'est à dire avec un tampon de lavage dont la force ionique est augmentée par rapport à la force ionique de l'étape a). Avantageusement, la force ionique du tampon de lavage est augmentée de 2 à 500 fois par rapport à la force ionique de l'étape a). Avantageusement, la force ionique du tampon de lavage est augmentée de 100 à 500 fois, de préférence de 200 à 500 fois, par rapport à la force ionique de l'étape a).

On a montré dans les exemples que l'utilisation à l'étape a') d'un tampon de lavage ayant une force ionique élevée, en particulier une concentration élevée en NaCl, permet d'éliminer efficacement les substances liées de manière non-spécifique au support d'affinité sans simultanément affecter de manière détectable la liaison du Facteur VII au support d'affinité.

A l'étape a'), on utilise ainsi de préférence un tampon de lavage ayant une concentration finale en NaCl d'au moins 1 M.

Selon l'invention, un tampon de lavage ayant une concentration finale en NaCl d'au moins 1 M englobe les tampons de lavage ayant une concentration finale en NaCl d'au moins 1,5 M, 2 M, 2,5 M ou au moins 3 M.

De préférence un tampon de lavage utilisé à l'étape a') du procédé a une concentration finale en NaCl d'au plus 3,5 M. Avantageusement, un tampon de lavage utilisé à l'étape a') du procédé a une concentration finale en NaCl comprise entre 1,5 et 3,5, de préférence entre 2 et 3,5, de préférence entre 2,5 et 3,5, de préférence entre 3 et 3,5.

On a également montré dans les exemples que l'utilisation à l'étape a') d'un tampon de lavage ayant une hydrophobicité élevée, en particulier une concentration élevée en propylène glycol, permet d'éliminer efficacement les substances liées de manière non-spécifique au support d'affinité sans simultanément affecter de manière détectable la liaison du Facteur VII au support d'affinité.

A l'étape a'), on utilise ainsi de préférence un tampon de lavage ayant une teneur finale en propylène glycol d'au moins 20%.

Selon l'invention, un tampon de lavage ayant une teneur finale en propylène glycol d'au moins 20% englobe les tampons de lavage ayant une teneur finale en propylène glycol d'au moins 25% M, 30%, 35%, 40% , 45%, 50%, 55%, ou au moins 60% en volume, par rapport au volume total du tampon de lavage.

De préférence un tampon de lavage utilisé à l'étape a') du procédé a une teneur finale en propylène glycol d'au plus 50%. Avantageusement, un tampon de lavage utilisé à l'étape a') du procédé a une teneur finale en propylène glycol comprise entre 20% et 50%, de préférence entre 30% et 50%.

Selon un mode de réalisation particulier, le tampon de lavage utilisé à l'étape a') contient à la fois du NaCl et du propylène glycol comme décrit ci-dessus.

L'étape b) consiste en une étape d'élution des molécules de la protéine plasmatique d'intérêt ayant formé des complexes avec les aptamères nucléiques au cours de l'étape a).

Comme cela est illustré dans les exemples, un avantage spécifique du procédé de purification ci-dessus est la possibilité de réaliser l'étape d'élution par mise en contact des complexes formés entre (i) les aptamères nucléiques immobilisés sur ledit support d'affinité et (ii) ladite protéine plasmatique avec un agent chélateur des ions divalents,

comme l'EDTA.

Cet avantage technique, qui est rendu possible, grâce aux caractéristiques du support d'affinité de l'invention, permet l'élution de la protéine plasmatique sans nécessiter un quelconque recours à l'utilisation de conditions drastiques d'élution, susceptibles de dénaturer, au moins partiellement, la protéine plasmatique d'intérêt. Lesdites condition drastiques qui sont évitées englobent l'utilisation d"un pH acide pour l'étape d'élution, ce qui est couramment pratiqué pour les procédés de purification de protéines sur les supports d'affinité connus, et tout particulièrement sur les supports d'affinité comprenant des anticorps immobilisés.

Ainsi, dans certains modes de réalisation du procédé de purification ci-dessus, l'étape b) est réalisée par mise en contact du support d'affinité avec un tampon d'élution contenant un agent chélateur des ions divalents, de préférence l'EDTA.

A titre illustratif, le tampon d'élution peut contenir une concentration finale d'EDTA d'au moins 1 mM et d'au plus 30 mM.

L'expression « au moins 1 mM » englobe au moins 2, 3, 4, 5, 6, 7, 8, 9 ou 10 mM.

L'expression « au plus 30 mM » englobe au plus 29, 28, 27, 26, 25, 24, 23, 22, 21, 20, 19, 18, 17, 16, 15, 14, 13, 12 ou 11 mM.

A l'étape c), on récupère la protéine plasmatique d'intérêt est purifiée en collectant le liquide d'éluat obtenu à la fin de l'étape b).

A la fin de l'étape c), on obtient une composition liquide purifiée de la protéine plasmatique d'intérêt. Ladite composition liquide purifiée peut ensuite être traitée de manière appropriée, selon toute technique connue de conditionnement et de conservation des protéines, y compris par flaconnage direct ou après dilution avec une solution adaptée, ou encore par lyophilisation, préférentiellement dans des conditions stériles et apyrogènes, puis conservation dans des conditions appropriées, à température ambiante, à -4°C ou bien à basse température, selon le type de conditionnement choisi.

Comme cela a déjà été mentionné précédemment dans la présente description, le support d'affinité de l'invention peut, avec les cycles successifs d'utilisation pour la purification d'une protéine plasmatique d'intérêt, subir une réduction de sa capacité d'absorption, par exemple du fait que l'étape c) d'élution ne permet pas systématiquement de libérer la totalité des molécules de protéine plasmatique, ce qui réduit le nombre de sites aptamères libres pour les cycles ultérieurs de purification.

Comme pour la totalité des supports de chromatographie connus, il est donc nécessaire, à des moments appropriés, de réaliser une étape de régénération du support d'affinité, afin de libérer la totalité des molécules de protéine plasmatique dudit support, et d'éliminer toute substance pouvant être liée au matériau solide du support d'affinité, en général par fixation non spécifique.

Ainsi, dans certains modes de réalisation, le procédé de purification de l'invention comprend une étape additionnelle d) de régénération du support d'affinité, par mise en contact dudit support d'affinité avec une solution de régénération.

Des tampons variés pour la régénération de support de chromatographie, en particulier de supports de chromatographie d'affinité sont bien connus par l'homme du métier, qui peuvent être utilisés à l'étape d) du procédé. L'homme du métier peut se référer par exemple à l'ouvrage de Mohr et al. (Affinity Chromatography: Practical and Theoretical Aspects, Peter Mohr, Klaus Pommerening, Edition: illustrated, CRC Press, 1985).

A titre illustratif, l'étape d) de régénération du support d'affinité peut être réalisée par mise en contact dudit support avec une solution tampon de Tris 50 mM, Ethylène Glycol 50%, comme cela est illustré dans les exemples.

Comme cela est illustré dans les exemples, le procédé de purification ci-dessus permet l'obtention d'une protéine plasmatique à un très haut degré de pureté, éventuellement à une degré de pureté supérieur à 99,95% en poids, par rapport au poids total des protéines contenues dans le produit final purifié.

Un autre avantage du procédé de purification ci-dessus, en particulier dans les modes de réalisation dans lesquels l'échantillon de départ consiste en un échantillon comprenant la protéine plasmatique humaine d'intérêt sous forme recombinante en mélange avec des protéines produites naturellement par le mammifère transgénique non humain, est que la composition finale comprenant la protéine humaine recombinante d'intérêt à haut degré de pureté est sensiblement exempte de protéines provenant dudit mammifère transgénique, et en particulier sensiblement exempte de protéines dudit mammifère, homologues de ladite protéine humaine recombinante.

A titre illustratif, on a montré dans les exemples que du facteur VII humain recombinant produit dans le lait d'une lapine transgénique, puis purifié avec le procédé de purification défini dans la présente description, comprend moins de 1,5% en poids des protéines dudit mammifère transgénique, par rapport au poids desdites protéines contenues initialement dans l'échantillon de départ. Dans la même exécution du procédé de purification selon l'invention, 85% en poids du facteur VII humain recombinant présent dans l'échantillon de départ était contenu dans le produit final à haut degré de pureté en Facteur VII humain recombinant, supérieur à 99,95% du poids des protéines présentes.

Il est décrit une composition purifiée d'une protéine plasmatique humaine recombinante comprenant au moins 99,9% en poids de ladite protéine humaine recombinante et qui est sensiblement exempte de protéines non humaines.

Il est décrit aussi une composition purifiée d'une protéine plasmatique humaine recombinante comprenant au moins 99,9% en poids de ladite protéine humaine recombinante et au plus 0,01 % en poids de protéines non humaines, les pourcentages en poids étant exprimés par rapport au poids total en protéines de adite composition purifiée.

Dans la composition purifiée ci-dessus, « au moins 99,9% » englobe au moins 99,91%, 99,92%, 99,93%, 99,94%, 99,95%, 99,96%, 99,97%, 99,98% et 99,99%.

Dans la composition purifiée ci-dessus, « au plus 0,01% » englobe au plus 0,09%, 0,08%, 0,07%, 0,06%, 0,05%, 0,04%, 0,03%, 0,02% et 0,01%..

Il est décrit aussi une composition purifiée telle que définie ci-dessus, utilisable comme médicament.

Il est également décrit une composition pharmaceutique comprenant une composition purifiée d'une protéine plasmatique humaine recombinante telle que définie ci-dessus, en combinaison avec un ou plusieurs excipients pharmaceutiquement acceptables.

La présente description décrit aussi à une composition purifiée telle que définie ci-dessus pour le traitement des troubles de la coagulation.

Il est également décrit l'utilisation d'une composition purifiée telle que définie ci-dessus pour la fabrication d'un médicament pour le traitement des troubles de la coagulation.

Sont décrits ci-dessous des modes de réalisation spécifiques d'aptamères se liant spécifiquement à une protéine de la coagulation, qui peuvent être avantageusement utilisés dans le procédé de purification selon l'invention.

Modes de réalisation spécifiques d'aptamères utilisables dans le procédé de purification selon l'invention.

La demanderesse a construit une famille d'aptamères nucléiques se liant spécifiquement aux protéines du plasma sanguin, et notamment au Facteur VII/VIIa humain dont elle a pu montrer l'existence de relations entre (i) les caractéristiques structurelles communes et (ii) la ou les caractéristiques fonctionnelles communes.

D'un point de vue structurel, la famille d'acides nucléiques, ou aptamères nucléiques, se liant spécifiquement au Facteur VII/VIIa humain et utilisables selon l'invention comprend au moins 15 nucléotides consécutifs d'un polynucléotide ayant au moins 40% d'identité en nucléotides avec l'acide nucléique de formule (I) suivante :
5'-[SEQ ID N°1]x-[SEQ ID N° X]-[SEQ ID N°2]y-3' (I), dans laquelle :

- « SEQ ID N° X » consiste en un acide nucléique choisi dans le groupe constitué des acides nucléiques de séquences SEQ ID N°3 à SEQ ID N°85 et SEQ ID N°87 à SEQ ID N°100,
- « x » est un entier égal à 0 ou 1, et
- « y » est un entier égal à 0 ou 1.

Selon certains aspects, l'acide de séquence SEQ ID N°X a une longueur de 15, 16, 17, 18, 19, 20, 21, 22, 23, 24, 25, 26, 27, 28, 29, 30, 31, 32, 33, 34, 35, 36, 37, 38, 39, 40,41, 42, 43, 44, 45, 46, 47, 48, 49, ou 50 nucléotides.

Selon d'autres aspects, l'acide nucléique de séquence SEQ ID N°X a une longueur de 43, 44, 45, 46, 47, 48 ou 49 nucléotides de longueur.

Selon certains autres aspects préférés, l'acide nucléique de séquence SEQ ID N°X a une longueur de 43, 44 ou 45 nucléotides de longueur.

Comme déjà mentionné précédemment, l'acide nucléique de formule (I) a au moins 15 nucléotides de longueur.

Dans certains aspects, l'acide nucléique de formule (I) a au moins 15, 16, 17, 18, 19, 20, 21, 22, 23, 24, 25, 26, 27, 28, 29, 30, 31, 32, 33, 34, 35, 36, 37, 38, 39, 40,41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80 ou 81 nucléotides de longueur, ce qui englobe les acides nucléiques possédant exactement chacune des longueurs spécifiées.

Dans certains aspects du procédé d'obtention des aptamères de formule (I), les cycles successifs de sélection réalisés pour construire la famille d'acides nucléiques d'intérêt se liant spécifiquement aux protéines du plasma sanguin ont abouti à isoler et caractériser, à chaque étape successive de sélection, des ensembles et sous-ensembles d'aptamères nucléiques comprenant, à leurs extrémités, 5' et 3', respectivement les séquences SEQ ID N°1 et SEQ ID N°2, encadrant structurellement une séquence variable SEQ ID N°X. Dans la famille principale d'aptamères nucléiques de l'invention, l'ensemble des séquences variables SEQ ID N°X ont, entre elles une identité de séquence en nucléotides d'au moins 40%. Cela signifie que, pour la séquence SEQ ID N° X, les contraintes de structure, pour conserver la propriété de liaison aux protéines du plasma sanguin, sont beaucoup moindres que les contraintes de structure pour les séquences localisées respectivement aux extrémités 5' et 3' de ces aptamères nucléiques.

Lorsque l'entier « x » est égal à 0 et l'entier « y » est égal à 1, les aptamères nucléiques de formule (I) englobent les acides nucléiques comprenant au moins 15 nucléotides consécutifs d'un polynucléotide ayant au moins 40% d'identité en nucléotides avec l'acide nucléique de formule (I-1) suivante
5' -[SEQ ID N° X]-[SEQ ID N°2] -3' (I-1),

Lorsque l'entier « x » est égal à 1 et l'entier « y » est égal à 0, les aptamères nucléiques de formule (I), englobent

les acides nucléiques comprenant au moins 15 nucléotides consécutifs d'un polynucléotide ayant au moins 40% d'identité en nucléotides avec l'acide nucléique de formule (I-2) suivante

5'-[SEQ ID N°1] -[SEQ ID N° X]-3' (I-2)

Lorsque l'entier « x » est égal à 0 et l'entier « y » est égal à 0, les aptamères nucléiques de formule (I), englobent les acides nucléiques comprenant au moins 15 nucléotides consécutifs d'un polynucléotide ayant au moins 40% d'identité en nucléotides avec l'acide nucléique de formule (I-3) suivante :

5'-[SEQ ID N° X]-3' (I-3)

Les aptamères nucléiques ci-dessus englobent donc les acides nucléiques comprenant au moins 15 nucléotides consécutifs d'un polynucléotide ayant au moins 40% d'identité en nucléotides avec un acide nucléique choisi dans le groupe constitué des acides nucléiques de séquences SEQ ID N°3 à SEQ ID N°85 et SEQ ID N°87 à SEQ ID N°100.

De manière générale, un premier polynucléotide ayant au moins 40% d'identité en nucléotides avec un second polynucléotide ou acide nucléique possède au moins 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% ou 100% d'identité en nucléotides avec ledit second polynucléotide ou acide nucléique.

Selon certains aspect d'un acide nucléique comprenant la séquence SEQ ID N° X, ladite séquence SEQ ID N° X est choisie dans le groupe constitué des acides nucléiques ayant au moins 15 nucléotides consécutifs d'une séquence possédant au moins 40% d'identité en nucléotides avec au moins l'une des séquences SEQ ID N° 3 à SEQ ID N° 85 et SEQ ID N°87 à SEQ ID N°100.

Selon certains aspects d'un acide nucléique comprenant la séquence SEQ ID N° X, ladite séquence SEQ ID N° X est choisie dans le groupe constitué des acides nucléiques possédant au moins 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% ou 100% d'identité en nucléotides avec au moins l'une des séquences SEQ ID N° 3 à SEQ ID N° 85 et SEQ ID N°87 à SEQ ID N°100.

Il résulte de ce qui précède que la présente description décrit une famille d'acides nucléiques monobrin ayant au moins 15 nucléotides consécutifs de l'enchaînement de formule (I) défini ci-dessus.

Le « pourcentage d'identité » entre deux séquences d'acides nucléiques, au sens de la présente invention, est déterminé en comparant les deux séquences alignées de manière optimale, à travers une fenêtre de comparaison.

La partie de la séquence nucléotidique dans la fenêtre de comparaison peut ainsi comprendre des additions ou des délétions (par exemple des « gaps ») par rapport à la séquence de référence (qui ne comprend pas ces additions ou ces délétions) de manière à obtenir un alignement optimal entre les deux séquences.

Le pourcentage d'identité est calculé en déterminant le nombre de positions auxquelles une base nucléique identique est observé pour les deux séquences comparées, puis en divisant le nombre de positions auxquelles il y a identité entre les deux bases nucléiques par le nombre total de positions dans la fenêtre de comparaison, puis en multipliant le résultat par cent afin d'obtenir le pourcentage d'identité en nucléotides des deux séquences entre elles.

L'alignement optimal des séquences pour la comparaison peut être réalisé de manière informatique à l'aide d'algorithmes connus.

De manière tout à fait préférée, le pourcentage d'identité de séquence est déterminé à l'aide du logiciel CLUSTAL W (version 1.82) les paramètres étant fixés comme suit : (1) CPU MODE = ClustalW mp ; (2) ALIGNMENT = « full » ; (3) OUTPUT FORMAT = « aln w/numbers » ; (4) OUTPUT ORDER = « aligned » ; (5) COLOR ALIGNMENT = « no » ; (6) KTUP (word size) = « default » ; (7) WINDOW LENGTH = « default » ; (8) SCORE TYPE = « percent » ; (9) TOPDIAG = « default » ; (10) PAIRGAP = « default » ; (11) PHYLOGENETIC TREE/TREE TYPE = « none » ; (12) MATRIX = « default » ; (13) GAP OPEN = « default » ; (14) END GAPS = « default » ; (15) GAP EXTENSION = « default » ; (16) GAP DISTANCES = « default » ; (17) TREE TYPE = « cladogram » et (18) TREE GRAP DISTANCES = « hide ».

La présente invention est en outre illustrée par les exemples suivants.

## EXEMPLES

### Exemple 1 : préparation d'un support d'affinité

Le support d'affinité a été réalisé à partir d'un matériau de support solide consistant en une matrice sur laquelle on a greffé de la streptavidine (streptavidin-agarose - Novagen®).

On a introduit un volume de 1 ml de gel dans une conteneur consistant en une colonne (i.d. 11mm). On a lavé le gel avec de l'eau purifiée, pour éliminer le solvant de stockage

Les caractéristiques du gel sont :

- *Capacité d'adsorption de biotine* : ≥ 85 nanomole / ml de gel
- *Test fonctionnel:* Capture >99% de thrombine biotinylée en 30 minutes à TA
- *Autres tests* : Protease-free , endo/exonucléase-free, RNase-free.
- *Conservateur :* 100 mM sodium phosphate pH7,5 + NaN3 0,02

La sortie de la colonne conditionnée (« packée ») (Hauteur de lit de gel = 1cm) est connectée à un détecteur d'absorbance équipé d'un filtre UV à 254 nm et d'un enregistreur.

Les aptamères nucléiques anti-FVII humain biotinylés de séquence SEQ ID N° 86 sont solubilisés dans de l'eau purifiée à la concentration finale de 0,5 mg/0,187 ml, soit une concentration molaire finale de 0,1 mM. La solution d'aptamères nucléiques a été activée à 95°C selon le cycle standard, pour l'immobilisation des aptamères sur le matériau support solide..

La solution d'aptamères nucléiques a été préalablement diluée par 4,8 ml d'eau purifiée puis 1,5 ml de tampon $Me^{++}$ (5 x concentré).

Le détecteur d'absorbance est ajusté à 1 AUFS (Absorbance Unit Full Scale) et on enregistre la DO à 254 nm de cette solution à 0,575 $UA_{254}$.

La solution d'aptamères nucléiques biotinylés est injectée sur le gel streptavidin-agarose pré-conditionnée (« prépackée ») et mise en recirculation avec une pompe péristaltique à un débit de 2,5 ml/minute, soit un temps de contact sur le gel de 24 secondes (entrée/sortie E/S). Dans ces conditions, la DO à 254 nm se stabilise rapidement à 0,05 $UA_{254}$, soit une valeur de 91% de couplage théorique, c'est-à-dire 0,455 mg d'aptamères nucléiques par millilitre de gel.

Un lavage avec un tampon 10 mM $CaCl_2$ + 4 mM MgCl2 puis en NaCl 2M est réalisé, afin d'éliminer les aptamères nucléiques qui ne sont pas fixées spécifiquement aux molécules de streptavidine greffées sur le matériau de support solide.

**Example 2 : Procédé de purification du Facteur VII recombinant humain**

Les supports d'affinité aptamères ont été testés à partir d'une préparation purifiée en FVII/FVIIa préparée selon la technique décrite dans la demande PCT n° WO2008/099077.

*Préparation de l'échantillon à purifier*

La matière biologique de départ est du lait de lapine transgénique contenant du FVII humain recombinant. La cassette d'expression comprend le transgène FVII humain placé sous le contrôle du promoteur du gène de la β-caseine.

Brièvement, 140 millilitres de lait ont été collectés sur 2 lapines en première lactation entre le Jour 4 et le Jour 12 après mise bas.

Le titre moyen en FVII amidolytique (FVII biologiquement activable) dans le lait collecté est de 928 UI/ml. Les laits sont conservés à une température de -80°C.

Pour l'essai, les laits de lapine sont décongelés au bain-marie à la température de 37°C, puis sont dilués par une solution de Citrate de Sodium pour une concentration finale en protéines de 62 g/l à un pH de 7,5.

Le traitement par le citrate de sodium permet la déstabilisation des micelles phosphocalciques de caséines.

La solution protéique riche en lipides du lait est ensuite clarifiée sur une séquence de filtres, respectivement (i) filtre profondeur de 15 à 0,5 μm de seuil de porosité puis (i) filtre membrane à 0,2 μm.

Un volume de 360 ml de solution filtrée ayant un titre en FVII de 198 UI/ml, soit 36 mg de FVII transgénique, est pré-purifié sur un gel de chromatographie MEP-HyperCel® (Pall BioSepra) d'un volume de 16 mL.. Ce gel de capture permet d'éliminer 95% des protéines du lait, dont la plus grande partie des caséines, tout en conservant 60% de la quantité de FVII initiale.

Une quantité de 17,5 mg de FVII de faible pureté (~5%) obtenu à la fin de l'étape ci-dessus est purifiée par chromatographie d'échanges d'ions, en utilisant un gel de Q-sepharose® XL (GE Healthcare) d'un volume de 20 mL, Le FVII humain est élué avec un volume de 78 ml de tampon comprenant 5 mM de chlorure de calcium. La concentration en FVII amydolitique est de 337 UI/ml, soit 0,17 mg de FVII/ml et la concentration en protéines totales est estimée à 0,18 mg/ml par mesure de DO à 280 nm et $\varepsilon^{1\%}$ = 13, soit une pureté en FVII de 94%.

Les protéines résiduelles provenant du lait de lapine sont difficilement séparables du FVII à ce stade, soit parce qu'il existe des homologies de structures telles que des protéines à GLA-domaines ou à domaine EGF, ou bien des homologies physico-chimiques (charge ionique et/ou taille moléculaire proche). Les techniques classiques permettent

une amélioration de la pureté jusqu'à 99,95% par des techniques orthogonales (combinaison de séparation sur gel d'hydroxyapatite et par chromatographie d'exclusion de taille). Cependant, pour l'injection répétée à l'homme, la charge en protéines exogènes admises pour les protéines de recombinaison génétique ne doivent pas dépasser 50 ppm soit une pureté > 99,995%. Une telle pureté ne semble atteignable qu'après purification sur matrice d'affinité.

*Etape de purification du FVII recombinant humain sur le support d'affinité de l'invention*

Un volume de 6 ml de la solution de FVII humain purifié (1,1 mg de FVII) obtenue à la fin de l'étape précédente est utilisée pour l'étape de purification à un haut niveau de pureté du FVII humain recombinant avec le support d'affinité de l'invention.

La solution de FVII obtenue à l'étape précédente, préalablement ajustée à 4 mM $MgCl_2$ et 10 mM $CaCl_2$ et pH 7,5, est injectée sur le gel Aptamère-agarose (support d'affinité) avec une pompe péristaltique à un débit de 0,1 ml/minute soit un temps de contact avec le support d'affinité de 10 minutes (E/S).

Après injection, le gel est lavé en tampon tris 50 mM + NaCl 50mM + 4 mM $MgCl_2$ + 10 mM $CaCl_2$ à pH 7,5.

Un volume de 10 ml de solution non adsorbé est collecté.

Le FVII est élué par un tampon tris 50mM + EDTA 10 mM à pH 7,5. La collecte du pic d'élution est réalisée suivant le profil de DO.

Selon les calculs molaires, la quantité d'aptamères nucléiques immobilisés dans le support d'affinité est de 17 nanomoles, ce qui correspond, pour une interaction mole à mole avec les molécules de FVII, à une capacité absolue du support d'affinité de 0,9 mg de FVII.

La figure 1 illustre un profil de chromatographie du FVII humain recombinant produit dans le lait de lapine, avec un suivi en continu des valeurs d'absorbance (D.O.) à 254 nanomètres.

Sur la Figure 1, l'inflexion (2) de la courbe d'absorption, après le moment de l'injection (1) illustre le début de la saturation du support d'affinité avec le FVII humain recombinant. Au temps (3) l'injection de FVII humain recombinant est stoppée. Pour illustrer l'échelle linéaire des temps sur la Figure 1, on indique que la durée entre le temps de début d'injection (1) et le temps de fin d'injection (2) est de 10 minutes. Le support d'affinité continue de se saturer avec la protéine de la coagulation d'intérêt : des complexes entre (i) les aptamères nucléiques anti-FVII du support d'affinité et (ii) les molécules de FVII humain recombinant initialement contenues dans la composition à purifier ont été formés. Après passage de la composition à purifier, on procède à une étape de lavage (6) de la colonne avec le tampon de lavage spécifié précédemment. Puis on réalise l'étape d'élution, par injection au temps (4) de la solution tampon comprenant une concentration finale de 10 mM en EDTA. Le pic d'absorption illustre la libération du FVII humain recombinant à partir des complexes Aptamères nucléiques/FVII recombinant. On note que les molécules de FVII humain recombinant sont libérées rapidement et donc dans un faible volume. En conséquence, grâce au support d'affinité, on obtient une solution d'élution de forte concentration en protéine FVII humain recombinant. Au temps (5), on a réalisé une étape de régénération du support d'affinité, avec un tampon Tris à 50 mM. Le pic d'absorbance visible en (7) correspond aux substances libérées du support d'affinité du fait de l'étape de régénération.

*Capacité dynamique d'absorption du support d'affinité*

Le tableau 1 ci-dessous présente les résultats de l'essai, qui montrent une capacité dynamique d'adsorption de 0,45 à 0,49 mg/ml des matrices d'affinité soit 50 à 55% de ligands bio-disponibles.

En EDTA, on calcule un rendement dynamique d'élution d'environ 75%.

Tableau 1 : bilan FVII recombinant humain et Protéines totales des essais de matrices Aptamères-aaarose:

| | **FVII recombinant** | | **Protéines (total mg)** | | **DBC (mg/m)l** | **DE(%)** |
|---|---|---|---|---|---|---|
| **Départ** | 2228 | 100% | 1,42 | 100% | | |
| | | | | | | |
| **Final** | | | | | | |
| Non adsorbé | 924 | 41% | 0,57 | 40% | | |
| éluat | 971 | 44% | 0,61 | 43% | 0,49 | 74% |

(suite)

| | FVII recombinant | | Protéines (total mg) | | DBC (mg/m)l | DE(%) |
|---|---|---|---|---|---|---|
| Résultats pondéraux | | 85% | | 83% | | |
| *Départ* : échantillon de départ<br>*Final* : composition des fractions<br>*BDC :* capacité dynamique d'absorption (pour « Dynamic Binding Capacity »)<br>*DE :* Rendement dynamique d'élution (pour « Dynamic Elution ») ; qui représente le rapport entre le FVII recombinant élué et le FVII recombinant adsorbé, exprimé en pourcentage | | | | | | |

*Capacité de séparation spécifique du support d'affinité*

Les supports d'affinité ont été évalués en spécificité par un dosage ELISA spécifique des protéines du lait de lapines.

Les résultats sont représentés dans le Tableau 2 ci-dessous.

Tableau 2 : bilan de la spécificité des supports d'affinité :

| | FVII recombinant (total mg) | | Protéines du lait de lapine (RMP) | | RMP (ppm) | % pureté FVII |
|---|---|---|---|---|---|---|
| **Départ** | 1,11 | 100% | 16992 | 100% | 16782 | 98,32% |
| | | | | | | |
| **Final** | | | | | | |
| Non adsorbé | 0,46 | 41% | 14590 | 40% | 34738 | 96,53% |
| éluat | 0,49 | 44% | 217 | 43% | 492 | 99,95% |
| Résultats pondéraux | | 85% | | 83% | | |

Les résultats du Tableau 2 ci-dessus montrent que l'on obtient une moyenne de 2 $log_{10}$ d'élimination des Aptamères-agarose portant la pureté du FVII humain transgénique de 98,3% à 99,95%. Ceci montre une bonne spécificité des aptamères vis-à-vis du FVII humain et très peu d'interactions aux protéines résiduelles du lait de lapines.

Une amélioration est possible par des lavages intermédiaires avant élution par des solutions telles que NaCl 2M et/ou Propylène Glycol ou Ethylène Glycol à 50% si dans ces conditions le FVII ne s'élue pas.

Les résultats de l'exemple 2 illustrent les excellentes caractéristiques des supports d'affinité sur gel Aptamère-agarose (Apta-2) avec une capacité dynamique d'adsorption d'au moins 1 mg de FVII par mg de ligand avec un rendement d'élution d'au moins 75%. La spécificité est également bien établie avec une nette amélioration de la pureté (~99,95%), une élimination de 2 $log_{10}$ des RMP « rabbit milk proteins » résiduelles. Le taux final s'établit à environ 500 ppm sur ces 2 essais non optimisés.

**Exemple 3 : Procédé de purification du Facteur IX humain plasmatique**

**A. Matériel et Méthodes**

A.1. Le support de chromatographie d'affinité

Matière Gel d'affinité Mapt-1,Sans spacer, densité de ligand théorique 0,46 mg/ml : volume 1 ml,

On fixe directement l'aptamère sur le matériau support de chromatographie, par une réaction de couplage chimique.

L'aptamère utilisé est l'aptamère de séquence SEQ ID N° 101

A.2. Le produit de départ

Le produit de départ consiste en une composition enrichie en Facteur IX issu de plasma humain, qui est commercialisée par le LFB sous la dénomination Betafact®.

Matière première injectée : Betafact MPVP (FIX plasmatique à 60 % pureté), Charge de 200 UI (soit 800 µg) de facteur IX par ml de gel, temps de contact 10 minutes.

A.3. Le protocole de purification

| | |
|---|---|
| Equilibration gel | Tris-HCl 0,050 M, CaCl2 0,010 M, pH 7,5, |
| Elution | Tris-HCl 0,020 M, EDTA 0,010 M, pH 7,5, |
| Régénération | Tris-HCl 0,020 M, NaCl 1 M, Propylène glycol 50 %, pH 7,5 |

On détecte les pics de protéines par mesure de la valeur d'absorbance à la longueur d'onde de 280 nanomètres.

A.4. Protocole d'analyse par électrophorèse sur gel SDS PAGE

Gels NOVEX (Invitrogen) 10 puits, 4-12%, Bis-Tris; tampon de migration MES, migration à 200V pendant 50min. Coloration CBB (G250) ou AgNO3 (kit GE)

**B. Résultats**

Les résultats sont illustrés sur les figures 2 et 3.

La Figure 2 représente la courbe des valeurs de mesure de l'absorbance à 280 nm en fonction du temps. Sur la Figure 2, le pic n°1 correspond à la fraction du produit de départ qui n'a pas été retenue sur la colonne. Le pic n°2 correspond à la fraction d'élution. Le pic n° 3 correspond à la fraction désorbée du support par la mise en oeuvre de l'étape de régénération..

La Figure 3 est un cliché d'un gel d'électrophorèse SDS PAGE. De la gauche vers la droite du gel sur la Figure 3 les pistes représentent les résultats de migration des produits de départ suivants :

- piste « MD » : la composition de départ Betafact®,
- piste « NR » : la fraction non retenue correspondant au pic n° 1 du profil chromatographique de la Figure 2
- piste « E1 » : la fraction d'élution correspondant au pic n°2 du profil chromatographique de la figure 2

Tableau 3

| Stade | % de FIX |
|---|---|
| Produit de départ | 51 % |
| Non retenu | 44% |
| Eluat | 100% |

Le tableau 3 récapitule les pourcentages de pureté du FIX obtenue par intégration du profil électrophorétique dans les différentes fractions. Le % de FIX est calculé selon un mode opératoire bien connu de l'homme du métier, par intégration quantitatives des densités des bandes électrophorétiques du gel coloré au CBB (données chiffrées correspondant à la figure 3). L'intégration quantitative des densités des bandes électrophorétiques peut être obtenue en scannant le gel avec un scanner adapté.

Les résultats représentés sur la Figure 3 et le tableau 3 confirment ceux de la figure 2. Ces résultats illustrent que le support de chromatographie sur lequel est immobilisé l'aptamère permet la purification spécifique du Facteur IX humain à partir d'un milieu complexe comme une fraction plasmatique enrichie en Facteur IX.

On peut conclure que l'éluat présente une bonne pureté electrophorétique et est caractérisé par un maintient de la fonctionnalité du FIX. Cette expérience montre la capacité de l'aptamère de séquence SEQ ID N° 101 directement couplé au support chromatographique, donc en l'absence de chaîne espaceur, à lier et à purifier du FIX dans un milieu complexe contenant des impuretés plasmatiques

## Exemple 4 : Procédé de purification du Facteur IX recombinant contenu dans un extrait de lait de truie transgénique

### A. Matériel et Méthodes

A.1. Le support de chromatographie d'affinité

Matière Gel d'affinité sur lequel est immobilisé l'aptamère Mapt-1,par couplage direct sans chaîne espaceur. Densité de ligand théorique 0,46 mg/ml : volume 1 ml,

On fixe directement l'aptamère sur le matériau support de chromatographie, par une réaction de couplage chimique.
L'aptamère utilisé est l'aptamère Mapt 1 de séquence SEQ ID N° 101

A.2. Le produit de départ

Le produit de départ consiste en un lait de truie transgénique clarifié et pré-purifié sur MEP HyperCel : 1,8% de pureté Echantillon dialysé contre le tampon d'adsorption / équilibration de la résine afin d'éliminer le citrate de sodium. Charge de 302 UI (soit 1200 $\mu$g) de facteur IX par ml.

A.3. Le protocole de purification

| | |
|---|---|
| Equilibration gel | Tris-HCl 0,050 M, CaCl2 0,010 M, pH 7,5, |
| Elution | Tris-HCl 0,020 M, EDTA 0,010 M, pH 7,5, |
| Régénération | Tris-HCl 0,020 M, NaCl 1 M, Propylène glycol 50 %, pH 7,5 |

L'échantillon est injecté avec un débit de 0,1ml/min pendant 10min, le gel est ensuite lavé pendant 5min à 0,5ml/min. L'élution et la régénération sont réalisés par injection de 2ml de chacun des tampons avec un débit de 0,5ml/min.

On détecte les pics de protéines par mesure de la valeur d'absorbance à la longueur d'onde de 280 nanomètres.

A.4. Protocole d'analyse par électrophorèse sur gel SDS PAGE

A.5. Protocole de mesure de l'activité spécifique en Facteur IX

La mesure de la quantité de FIX (mesure antigénique) a été réalisée avec le kit FIX Ag Serachrom 0943 (Stago) selon les recommandations du fournisseur.

La mesure de l'activité enzymatique du FIX a été réalisée par un test chromogénique avec le kit Biophen FIX réf. 221802 (Hyphen BioMed) selon les recommandations du fournisseur.

L'activité spécifique est calculée par le ratio suivant :
Activité enzymatique du FIX/Quantité de FIX

### B. Résultats

Les résultats sont illustrés sur les figures 4 et 5.

La Figure 4 représente la courbe des valeurs de mesure de l'absorbance à 280 nm en fonction du temps. Sur la Figure 4, le pic n°1 correspond à la fraction du produit de départ qui n'a pas été retenue sur la colonne. Le pic n°2 correspond à la fraction d'élution. Le pic n° 3 correspond à la fraction désorbée du support par la mise en oeuvre de l'étape de régénération..

Les résultats de la Figure 4 montrent que le pic d'élution est très étroit, ce qui illustre la très grande spécificité pour le Facteur IX humain du support de chromatographie sur lequel est immobilisé l'aptamère.

La Figure 5 est un cliché d'un gel d'électrophorèse SDS PAGE. De la gauche vers la droite du gel sur la Figure 5 les pistes représentent les résultats de migration des produits de départ suivants :

- piste « E5 » : la composition de départ de lait de truie transgénique contenant du Facteur IX humain transgénique pré-purifié par une étape chromatographique MEP HyperCel,

- piste « E6 » : la fraction non retenue correspondant au pic n° 1 du profil chromatographique de la Figure 4
- piste « E7 » : la fraction d'élution correspondant au pic n°2 du profil chromatographique de la figure 4
- piste « E8 » la fraction d'élution recueillie en aval du pic n°2 et an amont du pic n°3 du profil chromatographique de la figure 4
- piste « T FIX » : Témoin de facteur IX purifié.

Les résultats représentés sur la Figure 5 confirment ceux de la figure 4. Ces résultats illustrent que le support de chromatographie sur lequel est immobilisé l'aptamère permet la purification spécifique du Facteur IX humain à partir d'un milieu complexe comme une fraction plasmatique enrichie en Facteur IX.
De plus, les résultats de l'exemple illustrent le degré important d'enrichissement en Facteur IX humain qui est obtenu après passage du produit dé départ de composition complexe sur le support de chromatographie d'affinité sur lequel est immobilisé l'aptamère Mapt 2 de séquence SEQ ID N° 86.
On peut conclure que L'éluât présente une bonne pureté electrophorétique avec un gain de pureté important par rapport au produit de départ (> 26 fois). La seconde bande identifié dans l'éluat correspond certainement à une autre forme de FIX.

**Exemple 5 : Procédé de purification du Facteur VII plasmatique humain**

**A. Matériel et Méthodes**

A.1. Le support de chromatographie d'affinité

Matière Gel d'affinité sur lequel on a immobilisé l'aptamère « Mapt-2 core » couplé directement à la biotine, sans chaîne espaceur entre l'aptamère et la biotine. L'aptamère est immobilisé sur un gel streptavidine (Fournisseur Novagen) grâce à une biotine en 5' terminal avec une densité de ligand théorique 0,4 mg/ml : volume 1 ml,
**[0233]** L'aptamère utilisé est l'aptamère Mapt-2 Core de séquence SEQ ID N° 20

A.2. Le produit de départ

Le produit de départ consiste en une composition de Facteur VII plasmatique humain purifié à 98%..

A.3. Le protocole de purification

| | |
|---|---|
| Equilibration gel : | Tris-HCl 0,050 M, CaCl2 0,010 M, MgCl2 0,05mM pH 7,5, |
| Elution : | Tris-HCl 0,020 M, EDTA 0,010 M, pH 7,5, |

240µg de Facteur VII plasmatique humain purifié à 98% est injecté avec un débit de 0,5ml/min en tampon d'équilibration
Après détection du pic de non retenu 2 volumes de colonne de tampon d'élution est injecté.
On détecte les pics de protéines par mesure de la valeur d'absorbance à la longueur d'onde de 280 nanomètres.

**B. Résultats**

Les résultats sont illustrés sur la figure 6 et 7
La Figure 6 représente la courbe des valeurs de mesure de l'absorbance à 280 nm en fonction du temps. Sur la Figure 6, le pic n°1 correspond à la fraction du produit de départ qui n'a pas été retenue sur la colonne. Le pic n°2 correspond à la fraction d'élution.
Le produit de départ ainsi que le produit élué a été analysé en SDS PAGE avec coloration au nitrate d'argent afin de visualiser l'élimination des impuretés. La Figure 7 représente ce gel : la piste n°1 correspond à la fraction du produit de départ et la piste n°2 à la fraction d'élution. Malgré la pureté importante du produit de départ on constate que la fraction éluée ne contient plus de contaminant ou de formes dégradées.
Les résultats de la Figure 6 et 7 montrent que l'aptamère de séquence SEQ ID N° 20 est capable de lier le FVII humain et de l'éluer spécifiquement en présence d'EDTA.

**Exemple 6 : Absence de liaison de l'aptamère au FVII de lapin**

**A. Matériel et Méthodes**

A.1. Le support de chromatographie d'affinité

Gel d'affinité couplé à la streptavidine sur lequel on a immobilisé l'aptamère de séquence SEQ ID N° 101 par l'intermédiaire d'une chaîne espaceur (Fournisseur Novagen) via une biotine en 5' terminal avec une densité de ligand théorique 0,35 mg/ml : volume 1 ml,

L'aptamère utilisé est l'aptamère de séquence SEQ ID N° 86

A.2. Le produit de départ

Eluât d'hydroxyapatite enrichi en FVII de lapin obtenu par purification à partir de plasma de lapin, temps de contact 10 minutes, débit 0,5ml/min

A.3. Le protocole de purification

| | |
|---|---|
| Equilibration gel | Tris-HCl 0,050 M, CaCl2 0,010 M, MgSO3 0,05mM pH 7,5, |
| Elution | Tris-HCl 0,050 M, EDTA 0,010 M, pH 7,5, |

36μg injecté dans le gel avec temps de contact 10 minutes, l'élution est réalisée par injection de 2ml de tampon d'élution

On détecte les pics de protéines par mesure de la valeur d'absorbance à la longueur d'onde de 280 nanomètres.

A.4. les protocoles d'analyse des fractions en protéines et en Facteur VII

Les fractions sont analysées pour leur activité amidolytique en dosage chromogénique grâce à un kit Stago selon les recommandations du fournisseur (Kit Facteur VIIa StatClot). L'activité amidolytique est ensuite convertie en μg de FVII contenu dans ladite fraction.

**B. Résultats**

Les résultats sont illustrés sur le Tableau 4 ci-dessous.

Tableau 4

| Etapes | Protéines (mg/ml) | FVIIam (UI/ml) | Volume (ml) | Quantité FVII (μg) | Pureté (%) | Rdt étape (%) |
|---|---|---|---|---|---|---|
| **Starting material** | 1.38 | 57 | 2.5 | 71 | 2% | 100% |
| **Eluat Dialysé** | 0.97 | 21.8 | 3.4 | 36.7 | 1% | 52% |
| **Eluat Mapt-2** | NA | 0.04 | 4.0 | 0.08 | NA | 0.2% |

Les résultats du tableau 4 montrent que le Facteur VII de lapin n'est pas retenu sur le gel d'affinité sur lequel est immobilisé l'aptamère Mapt-2.

**Exemple 7 : Modes de réalisation spécifiques d'un protocole d'interaction du Facteur VII humain avec un aptamère sur Biacore (résistance au NaCl)**

**A. Matériel et Méthodes**

On a fabriqué un support solide sur lequel ont été immobilisées des molécules de l'aptamère nucléique de séquence SEQ ID N° 86, aussi désigné ici « Mapt2 ». Préalablement à sa fixation sur le support solide, l'extrémité 5'

de l'aptamère Mapt2 a été chimiquement couplée à une chaîne espaceur constituée de 4 molécules de PEG(C18). Puis, l'extrémité libre de la chaîne espaceur, opposée à l'extrémité couplée à l'aptamère, a été couplée à une molécule de biotine.

On dispose d'un support solide contenant des molécules de streptavidine immobilisées (Serie S sensor Chip SA, GE)

Puis, on a mis en contact le support solide ci-dessus avec les composés aptamères ci-dessus afin d'immobiliser les acides nucléiques de séquence SEQ ID N° 86, par association non covalente entre les molécules de streptavidine du support et les molécules de biotine des composés aptamères.

L'aptamère Mapt2 est ainsi immobilisé avec un taux d'immobilisation de 3772 RU (1 RU correspond approximativement à 1 pg de produit immobilisé par $mm^2$).

Du FVII humain transgénique purifié obtenu à partir de lait de lapine transgénique (FVII HPTG, pureté : 98%) a été dilué dans du tampon de course (Tris 50 mM, NaCl 50 mM, $CaCl_2$ 10 mM, $MgCl_2$ 4 mM, pH 7,4) de manière à obtenir un échantillon de concentration 200mM en FVII.

L'échantillon a été injecté sur la puce (support solide) contenant l'aptamère Mapt2 immobilisé par une interaction biotine-streptavidine. Ensuite, des tampons de concentration croissante en NaCl ont été injectés sur le support solide (3 séries d'injection allant de 1 M de NaCl à 3 M de NaCl). Toutes les injections ont été réalisées avec un débit 30μl/min pendant 60 sec après l'injection. Après les 3 séries d'injection avec les 3 tampons contenant du NaCl, du tampon d'élution (EDTA, 10mM) a ensuite été injecté pendant 75 sec avec un débit de 30μl/min pour décrocher le FVII HPTG de l'aptamère.

Ces analyses sont effectuées avec l'appareil de RPS Biacore T100 (GE). La modélisation des interactions enregistrées sont effectuée à l'aide du Logiciel Biaevaluation (GE).

Les courbes de liaison de l'aptamère immobilisé Mapt2 au FVII humain transgénique ont été calculées avec le module dédié du logiciel Biacore® control Software, version 1.2.

Les résultats liaison de l'aptamère Mapt2 au FVII humain ont permis de déterminer que la liaison de l'aptamère Mapt2 au FVII humain n'est pas altérée par l'injection des tampons contenant du NaCl.

### B. Résultats

Les résultats sont illustrés dans la figure 8

## Exemple 8 : Modes de réalisation spécifiques d'un protocole d'interaction du Facteur VII humain avec un aptamère sur Biacore (résistance au propylène glycol)

### A. Matériel et Méthodes

On a fabriqué un support solide sur lequel ont été immobilisées des molécules de l'aptamère nucléique de séquence SEQ ID N° 86, aussi désigné ici « Mapt2 ». Préalablement à sa fixation sur le support solide, l'extrémité 5' de l'aptamère Mapt2 a été chimiquement couplée à une chaîne espaceur constituée de 4 molécules de PEG(C18). Puis, l'extrémité libre de la chaîne espaceur, opposée à l'extrémité couplée à l'aptamère, a été couplée à une molécule de biotine.

On dispose d'un support solide contenant des molécules de streptavidine immobilisées (Serie S sensor Chip SA, GE)

Puis, on a mis en contact le support solide ci-dessus avec les composés aptamères ci-dessus afin d'immobiliser les acides nucléiques de séquence SEQ ID N° 86, par association non covalente entre les molécules de streptavidine du support et les molécules de biotine des composés aptamères.

L'aptamère Mapt2 est ainsi immobilisé avec un taux d'immobilisation de 5319 RU (1 RU correspond approximativement à 1 pg de produit immobilisé par $mm^2$).

Du FVII humain transgénique purifié obtenu à partir de lait de lapine transgénique (FVII HPTG, pureté : 98%) a été dilué dans du tampon de course (Tris 50 mM, $CaCl_2$ 10 mM, $MgCl_2$ 4 mM, pH 7,4) de manière à obtenir un échantillon de concentration 200mM en FVII.

L'échantillon a été injecté sur la puce (support solide) contenant l'aptamère Mapt2 immobilisé par une interaction biotine-streptavidine. Ensuite, un tampon contenant 50% de propylène glyco a été injecté sur le support solide. Toutes les injections ont été réalisées avec un débit 30μl/min pendant 60 sec après l'injection. Après l'injection avec le tampon contenant 50% de propylène glycol, du tampon d'élution (EDTA, 10mM) a ensuite été injecté pendant 75 sec avec un débit de 30μl/min pour décrocher le FVII HPTG de l'aptamère.

Ces analyses sont effectuées avec l'appareil de RPS Biacore T100 (GE). La modélisation des interactions enregistrées sont effectuée à l'aide du Logiciel Biaevaluation (GE).

Les courbes de liaison de l'aptamère immobilisé Mapt2 au FVII humain transgénique ont été calculées avec le module dédié du logiciel Biacore® control Software, version 1.2.

Les résultats liaison de l'aptamère Mapt2 au FVII humain ont permis de déterminer que la liaison de l'aptamère Mapt2 au FVII humain n'est pas altérée par l'injection du tampon contenant du propylène glycol.

## B. Résultats

Les résultats sont illustrés dans la figure 9.

**Revendications**

1. Procédé pour la purification d'une protéine plasmatique humaine recombinante à partir d'un milieu de départ qui est une solution biologique provenant d'un animal non-humain transgénique pour ladite protéine plasmatique humaine, comprenant les étapes suivantes :

    a) mettre en contact un échantillon contenant la protéine plasmatique humaine avec un support d'affinité comprenant un support solide sur lequel sont immobilisés des aptamères désoxyribonucléiques se liant spécifiquement à ladite protéine plasmatique humaine, afin de former des complexes entre (i) les aptamères nucléiques immobilisés sur ledit support d'affinité et (ii) ladite protéine plasmatique humaine, et
    b) libérer la protéine à partir des complexes formés à l'étape a), et
    c) récupérer ladite protéine plasmatique humaine sous une forme purifiée.

2. Procédé selon la revendication 1 **caractérisé en ce qu'**il comprend une étape a') dans laquelle ledit support d'affinité est lavé avec un tampon de lavage.

3. Procédé selon la revendication 2 dans lequel le tampon de lavage est choisi parmi :

    - une solution tampon comprenant une concentration finale en NaCl d'au moins 1M, et
    - une solution tampon ayant une teneur finale en propylène d'au moins 20% (v/v).

4. Procédé selon l'une quelconque des revendication 1 à 3 , **caractérisé en ce que** la solution biologique comprend une protéine du plasma sanguin non humain qui est homologue à ladite protéine du plasma sanguin humaine.

5. Procédé selon la revendication 4, **caractérisé en ce que** ladite protéine du plasma sanguin humaine est l'homologue de ladite protéine du plasma non-humaine.

6. Procédé selon l'un des revendications 1 à 5, **caractérisé en ce que** l'étape b) est réalisée par mise en contact du support d'affinité avec un tampon d'élution contenant un agent chélateur des ions divalents.

7. Procédé selon la revendication 6, **caractérisé en ce que** l'agent chélateur des ions divalents est l'EDTA.

8. Procédé selon la revendication 7, dans lequel le tampon d'élution a une concentration d'au moins 1 mM et d'au plus 30 mM en EDTA.

9. Procédé selon l'une quelconque des revendications 1-8, dans lequel le milieu de départ est un fluide corporel d'un mammifère non-humain transgénique pour ladite protéine du plasma humain.

10. Procédé selon la revendication 9 dans lequel ledit fluide corporel est un lait ou une fraction du lait dudit mammifère non-humain transgénique.

11. Procédé selon l'une quelconque des revendications 1 à 10 dans laguelle la protéine plasmatique humaine est une protéine de la coagulation choisie parmi le groupe constitué par l'albumine, alpha/macroglobuline, antichymotrypsine, antithrombine, antitrypsine, Apo A, Apo B, Apo C, Apo D, Apo E, Apo F, Apo G, beta XIIa, C 1-inhibiteur, protéine C-réactive, C7, C1r, C1s, C2 C3, C4, C4bP, C5, C6, C1q, C8, C9, carboxypeptidase N, céruloplasmine, Facteur B, Facteur D, Facteur H, Facteur I, Facteur IX, Facteur V, Facteur VII, Facteur VIIa, Facteur VIII, Facteur X, Facteur XI, Facteur XII, Facteur XIII, Fibrinogène, Fibronectine, Haptoglobine, Hemopexine, Héparine cofacteur II, Histidine rich GP, IgA, IgD, IgE, IgG, ITI, IgM, Kininase II, Kininogène HPM, Lysozyme, PAI 2, PAI I, PCI, Plasmine, Inhibiteur de la plasmine, Plasminogène, Préalbumine, Prokallicréine, Properdine, Protéase Nexine INH, Protéine C, Protéine S, Protéine Z, Prothrombine, TFPI, Thiol-protéinase, Thrombomoduline, Facteur tissulaire (TF), TPA, Transcolabamine II, Transcortine, Transferrine, Vitronectine, et le Facteur de Willebrand

**12.** Procédé selon l'une quelconque des revendications 1 à 11 dans lequel la protéine plasmatique humaine est une protéine de la coagulation à activité enzymatique.

**13.** Procédé selon l'une quelconque des revendications 1 à 12 dans lequel le support solide du support d'affinité est une résine pour colonne de chromatographie d'affinité.

**14.** Procédé selon l'une quelconque des revendications 1 à 13 dans lequel lesdits aptamères désoxyribonucléiques sont inclus dans la structure d'un composé de formule (I) suivante

$$[FIX]_x\text{-}[SPAC]_y\text{- }[APT] \qquad (I),$$

dans laquelle :

- [FIX] signifie un composé d'immobilisation sur un support,
- [SPAC] signifie une chaîne espaceur,
- [APT] signifie un acide désoxyribonucléique se liant spécifiquement à une protéine plasmatique, aussi désigné aptamère désoxyribonucléique,
- x est un entier égal à 0 ou 1, et
- y est un entier égal à 0 ou 1.

**15.** Procédé selon l'une des revendications 4-14 dans lequel l'aptamère désoxyribonucléique est **caractérisé en ce que** : Kd humain/Kd non-humain < 0,01 où

- « Kd humain » est la constante de dissociation dudit aptamère pour ladite protéine plasmatique humaine, exprimée en unités molaires, et
- « Kd non-humain » est la constante de dissociation dudit aptamère pour la protéine homologue non humaine, exprimée dans les mêmes unités molaires.

**16.** Procédé selon l'une quelconque des revendications 1 à 15 dans lequel la protéine plasmatique humaine sous une forme purifiée obtenue à l'étape c) est mise en flaconnage direct, éventuellement après dilution avec une solution adaptée ou lyophilisation.

**Patentansprüche**

**1.** Verfahren zur Reinigung eines rekombinanten humanen Plasmaproteins aus einem Ausgangsmedium, das eine biologische Lösung ist, die von einem für besagtes humanes Plasmaprotein transgenen nicht-humanen Tier stammt, umfassend die folgenden Schritte:

a) Inkontaktbringen einer das humane Plasmaprotein enthaltenden Probe mit einem Affinitätsträger, umfassend einen festen Träger, auf dem Desoxyribonukleinsäure-Aptamere immobilisiert sind, die spezifisch besagtes humanes Plasmaprotein binden, um Komplexe zwischen (i) den Nukleinsäure-Aptameren, die auf besagtem Affinitätsträger immobilisiert sind, und (ii) besagtem humanen Plasmaprotein zu bilden, und
b) Freisetzen des Proteins aus den in Schritt a) gebildeten Komplexen, und
c) Wiedergewinnen besagten humanen Plasmaproteins in einer gereinigten Form.

**2.** Verfahren gemäß Anspruch 1, **dadurch gekennzeichnet, dass** es einen Schritt a') umfasst, in dem besagter Affinitätsträger mit einem Waschpuffer gewaschen wird.

**3.** Verfahren gemäß Anspruch 2, wobei der Waschpuffer ausgewählt ist aus:

- einer Pufferlösung, umfassend eine NaCl-Endkonzentration von wenigstens 1 M, und
- einer Pufferlösung mit einem Endpropylengehalt von wenigstens 20% (v/v).

**4.** Verfahren gemäß einem der Ansprüche 1 bis 3, **dadurch gekennzeichnet, dass** die biologische Lösung ein nicht-humanes Blutplasmaprotein umfasst, das zu besagtem humanen Blutplasmaprotein homolog ist.

**5.** Verfahren gemäß Anspruch 4, **dadurch gekennzeichnet, dass** besagtes humanes Blutplasmaprotein zu besagtem nicht-humanen Plasmaprotein homolog ist.

**6.** Verfahren gemäß einem der Ansprüche 1 bis 5, **dadurch gekennzeichnet, dass** der Schritt b) durch Inkontaktbringen des Affinitätsträgers mit einem Elutionspuffer durchgeführt wird, der einen Chelatbildner für zweiwertige Ionen enthält.

**7.** Verfahren gemäß Anspruch 6, **dadurch gekennzeichnet, dass** der Chelatbildner für zweiwertige Ionen EDTA ist.

**8.** Verfahren gemäß Anspruch 7, wobei der Elutionspuffer eine EDTA-Konzentration von wenigstens 1 mM und höchstens 30 mM besitzt.

**9.** Verfahren gemäß einem der Ansprüche 1 bis 8, wobei das Ausgangsmedium eine Körperflüssigkeit eines für besagtes humanes Plasmaprotein transgenen nicht-humanen Säugetiers ist.

**10.** Verfahren gemäß Anspruch 9, wobei besagte Körperflüssigkeit eine Milch oder eine Milchfraktion besagten transgenen nicht-humanen Säugetiers ist.

**11.** Verfahren gemäß einem der Ansprüche 1 bis 10, wobei das humane Plasmaprotein ein Koagulationsprotein ist, ausgewählt aus der Gruppe, bestehend aus Albumin, alpha-Makroglobulin, Antichymotrypsin, Antithrombin, Antitrypsin, Apo A, Apo B, Apo C, Apo D, Apo E, Apo F, Apo G, beta-XIIa, C1-Hemmer, C-reaktivem Protein, C7, C1r, C1s, C2, C3, C4, C4bP, C5, C6, C1q, C8, C9, Carboxypeptidase N, Caeruloplasmin, Faktor B, Faktor D, Faktor H, Faktor I, Faktor IX, Faktor V, Faktor VII, Faktor VIIa, Faktor VIII, Faktor X, Faktor XI, Faktor XII, Faktor XIII, Fibrinogen, Fibronectin, Haptoglobin, Hämopexin, Heparin-Cofaktor II, Histidinreichem GP, IgA, IgD, IgE, IgG, ITI, IgM, Kinase II, hochmolekularem Kininogen, Lysozym, PAI 2, PAI 1, PCI, Plasmin, Plasmin-Hemmer, Plasminogen, Präalbumin, Präkallikrein, Properdin, Protease-Nexin PNI, Protein C, Protein S, Protein Z, Prothrombin, TFPI, Thiol-Protease, Thrombomodulin, Gewebe-Faktor (TF), TPA, Transcobalamin II, Transcortin, Transferrin, Vitronectin und von-Willebrand-Faktor.

**12.** Verfahren gemäß einem der Ansprüche 1 bis 11, wobei das humane Plasmaprotein ein Koagulationsprotein mit enzymatischer Aktivität ist.

**13.** Verfahren gemäß einem der Ansprüche 1 bis 12, wobei der feste Träger des Affinitätsträgers ein Harz für eine Affinitätschromatographie-Säule ist.

**14.** Verfahren gemäß einem der Ansprüche 1 bis 13, wobei besagte Desoxyribonukleinsäure-Aptamere in der Struktur einer Verbindung folgender Formel (I) enthalten sind

$$[FIX]_x\text{-}[SPAC]_y\text{-}[APT] \qquad (I),$$

in der:

- [FIX] eine Verbindung zur Immobilisierung auf einem Träger bedeutet,
- [SPAC] eine Spacer-Kette bedeutet,
- [APT] eine Desoxyribonukleinsäure bedeutet, die spezifisch ein Plasmaprotein bindet und auch als Desoxyribonukleinsäure-Aptamer bezeichnet wird,
- x eine ganze Zahl gleich 0 oder 1 ist, und
- y eine ganze Zahl gleich 0 oder 1 ist.

**15.** Verfahren gemäß einem der Ansprüche 4 bis 14, wobei das Desoxyribonukleinsäure-Aptamer **dadurch gekennzeichnet ist, dass**:
Kd human/Kd nicht-human < 0,01, wo

- "Kd human" die Dissoziationskonstante besagten Aptamers für besagtes humanes Plasmaprotein, ausgedrückt in molaren Einheiten, ist, und
- "Kd nicht-human" die Dissoziationskonstante besagten Aptamers für besagtes nicht-humanes homologes Protein, ausgedrückt in denselben molaren Einheiten, ist.

**16.** Verfahren gemäß einem der Ansprüche 1 bis 15, wobei das in Schritt c) in gereinigter Form erhaltene humane Plasmaprotein in Flaschen direkt abgefüllt wird, gegebenenfalls nach Verdünnung mit einer geeigneten Lösung oder Lyophilisation.

## Claims

**1.** A method for purifying a recombinant human plasma protein being from a starting medium which is a biological solution from a nonhuman animal being transgenic for said human plasma protein, comprising the following steps:

a) bringing a sample containing a human plasma protein into contact with an affinity substrate comprising a solid substrate on which deoxyribonucleic aptamers which bind specifically to said human plasma protein are immobilized, in order to form complexes between (i) the deoxyribonucleic aptamers immobilized on said affinity substrate and (ii) said human plasma protein, and
b) releasing the protein from the complexes formed in step a), and
c) recovering said human plasma protein in a purified form.

**2.** The method according to claim 1, **characterized in that** it comprises a step a') wherein said affinity substrate is washed with a washing buffer.

**3.** The method according to claim 2 wherein the washing buffer is selected from:

- a buffer solution comprising a final NaCl concentration of at least 1M, and
- a buffer solution having a final propylene content of at least 20% (v/v).

**4.** The method according to any one claims 1 to 3, **characterized in that** the biological solution comprises a nonhuman blood plasma protein which is homologous to said human blood plasma protein.

**5.** The method according to claim 4, **characterized in that** said human blood plasma protein is the homologous to said nonhuman plasma protein.

**6.** The method according to claims 1 to 5, **characterized in that** step b) is carried out by bringing the affinity substrate into contact with an elution buffer containing a divalent-ion-chelating agent.

**7.** The method according to claim 6, **characterized in that** said divalent-ion-chelating agent is EDTA.

**8.** The method according to claim 7, wherein the elution buffer has an EDTA concentration of at least 1 mM and at the most of 30 mM.

**9.** The method according to any one of claims 1-8, wherein the starting medium is a body fluid from a nonhuman mammal being transgenic for said human plasma protein.

**10.** The method according to claim 9 wherein said body fluid is milk or a milk fraction from said transgenic nonhuman mammal.

**11.** The method according to any one of claims 1 to 10 wherein the human plasma protein is a coagulation protein selected from the group consisting of albumin, alpha/macroglobulin, antichymotrypsin, antithrombin, antitrypsin, Apo A, Apo B, Apo C, Apo D, Apo E, Apo F, Apo G, beta XIIa, CI-inhibitor, C-reactive protein, C7, C1r, C1s, C2, C3, C4, C4bP, C5, C6, C1q, C8, C9, carboxypeptidase N, ceruloplasmin, Factor B, Factor D, Factor H, Factor I, Factor IX, Factor V, Factor VII, Factor VIIa, Factor VIII, Factor X, Factor XI, Factor XII, Factor XIII, fibrinogen, fibronectin, haptoglobin, hemopexin, heparin cofactor II, histidine-rich GP, IgA, IgD, IgE, IgG, ITI, IgM, kininase II, kininogen HPM, lysozyme, PAI 2, PAI 1, PCI, plasmin, plasmin inhibitor, plasminogen, prealbumin, prokallikrein, properdin, protease nexin INH, protein C, protein S, protein Z, prothrombin, TFPI, thiol-proteinase, thrombomodulin, tissue factor (TF), TPA, transcolabamin II, transcortin, transferrin, vitronectin and von Willebrand factor.

**12.** The method according to any one of claims 1 to 11 wherein the human plasma protein is a coagulation protein with an enzymatic activity.

**13.** The method according to any one of claims 1 to 12 wherein the solid substrate material of the affinity substrate is a resin for an affinity chromatography column.

**14.** The method according to any one of claims 1 to 13 wherein said deoxyribonucleic aptamers are included in the structure of a compound of formula (I) below:

$$[FIX]_x\text{-}[SPAC]_y\text{-}[APT] \qquad (I)$$

in which:

- [FIX] means a compound for immobilization on a substrate,
- [SPAC] means a spacer chain,
- [APT] means a deoxyribonucleic acid which binds specifically to a plasma protein, also named deoxyribonucleic aptamer,
- x is an integer equal to 0 or 1, and
- y is an integer equal to 0 or 1.

**15.** The method according to any one of claims 4-14 wherein said deoxyribonucleic aptamer is **characterized in that**: human Kd/nonhuman Kd < 0,01 wherein

- "human Kd" is the dissociation constant of said aptamer for said human plasma protein, expressed in molar units, and
- "nonhuman Kd" is the dissociation constant of said aptamer for said nonhuman homologous protein, expressed in the same molar units.

**16.** The method according to any of claims 1 to 15 wherein said human plasma protein in a purified form obtained in step c) is set to direct bottling, optionally after a dilution with an appropriate solution or freeze-drying.

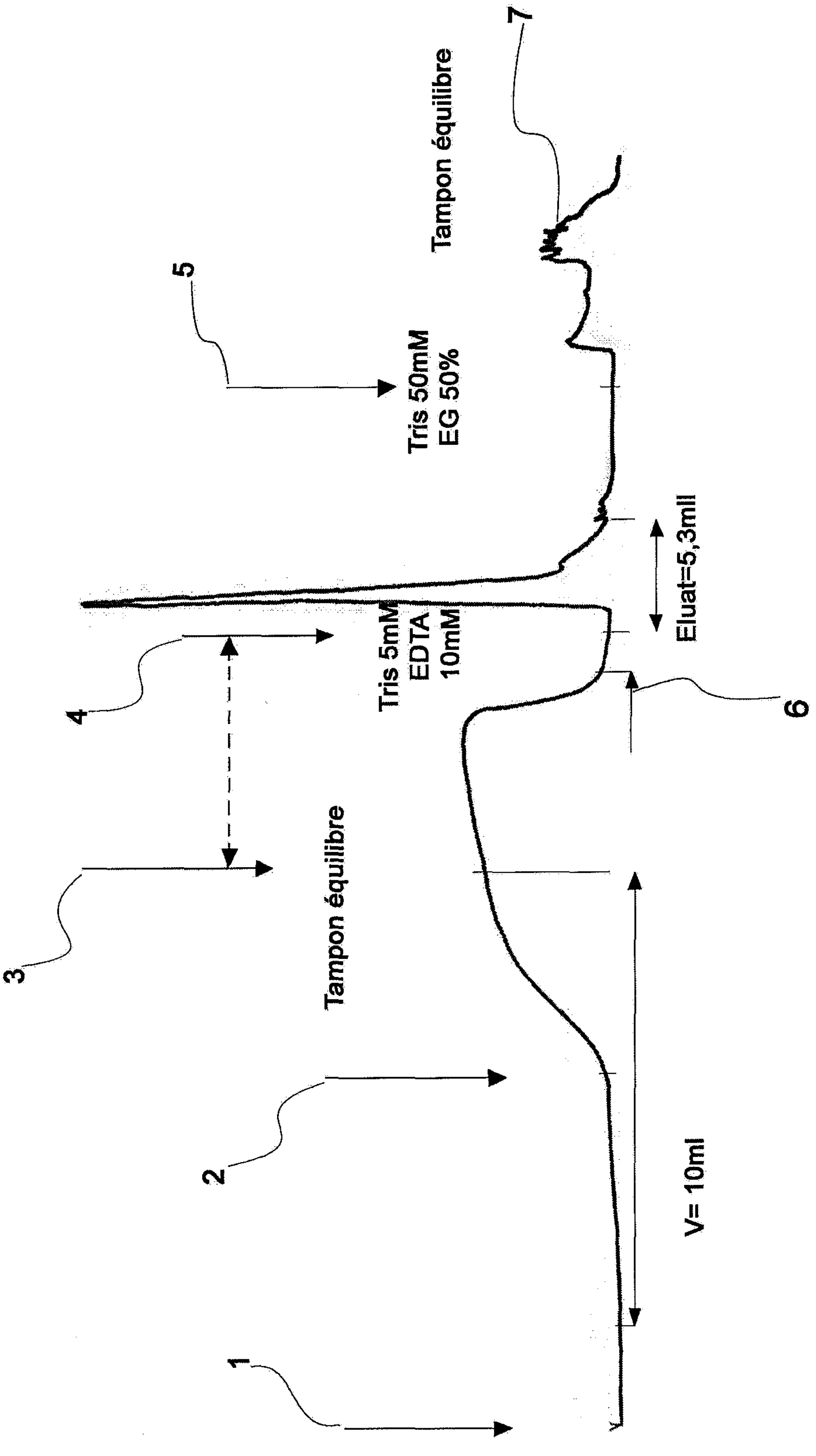
1
2
3
4
5
6
7
Tampon équilibre
Tris 5mM
EDTA
10mM
Eluat=5,3ml
Tris 50mM
EG 50%
Tampon équilibre
V= 10ml

Figure 1

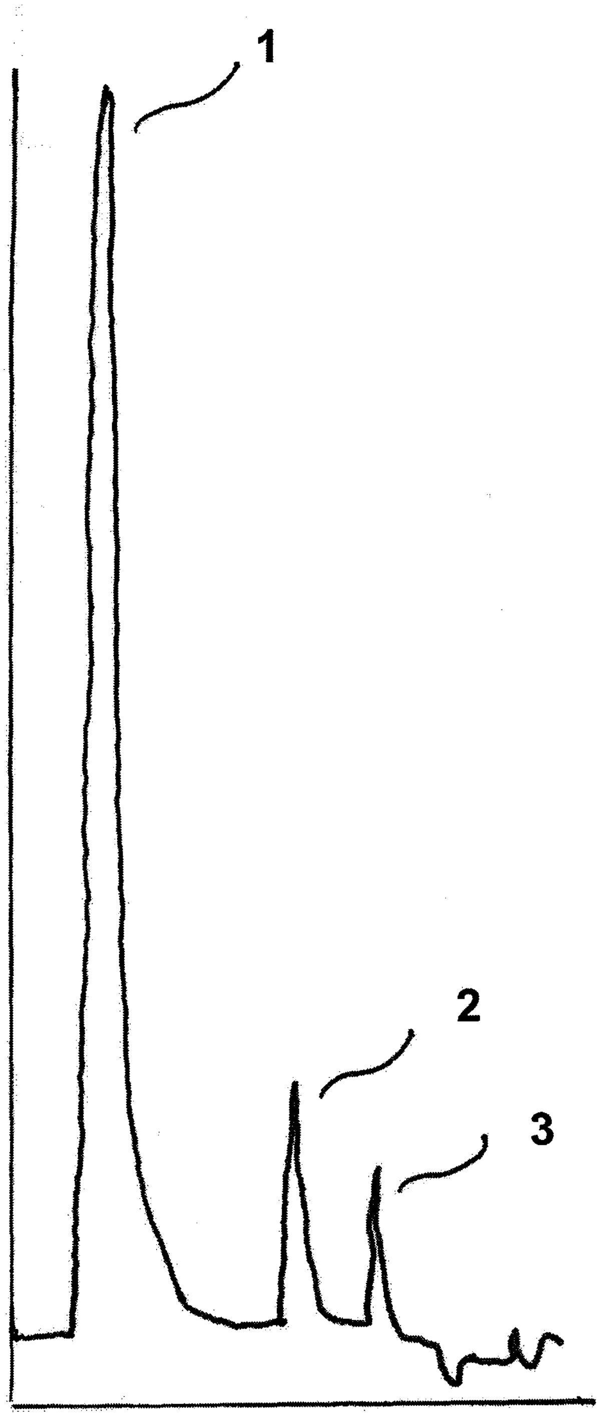


Figure 2

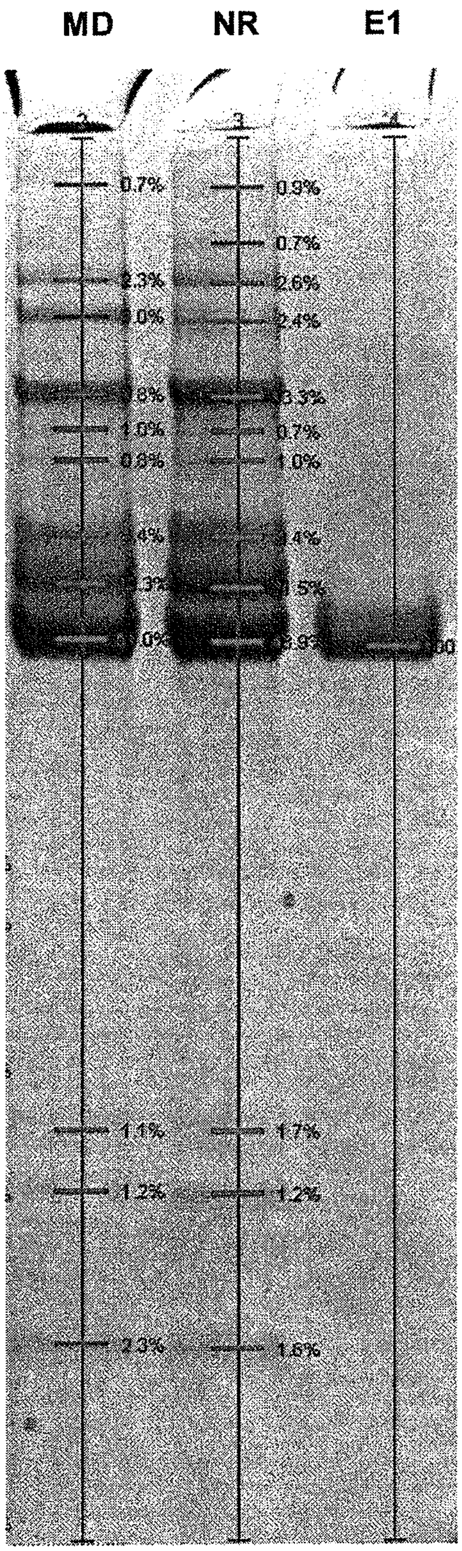


Figure 3

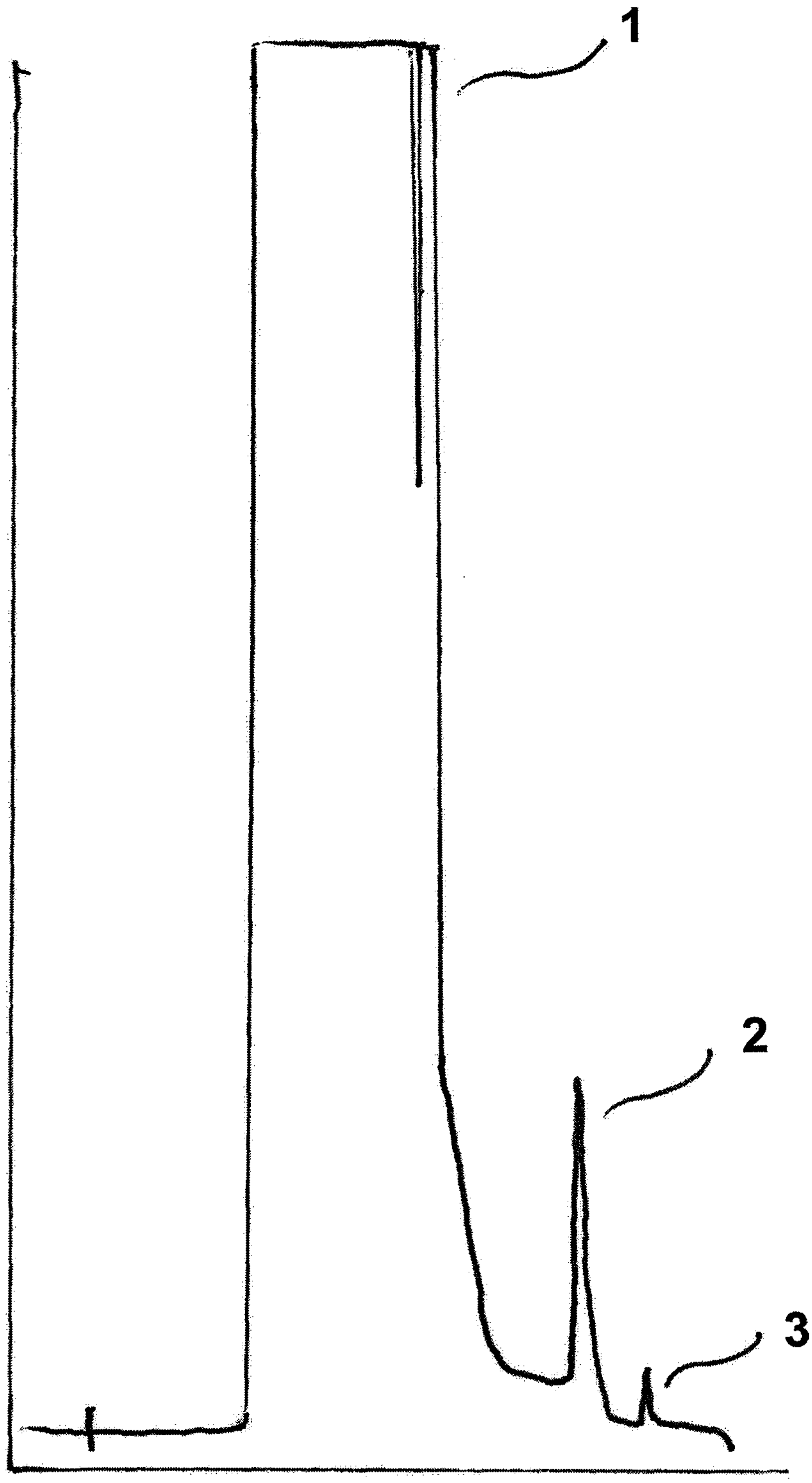


Figure 4

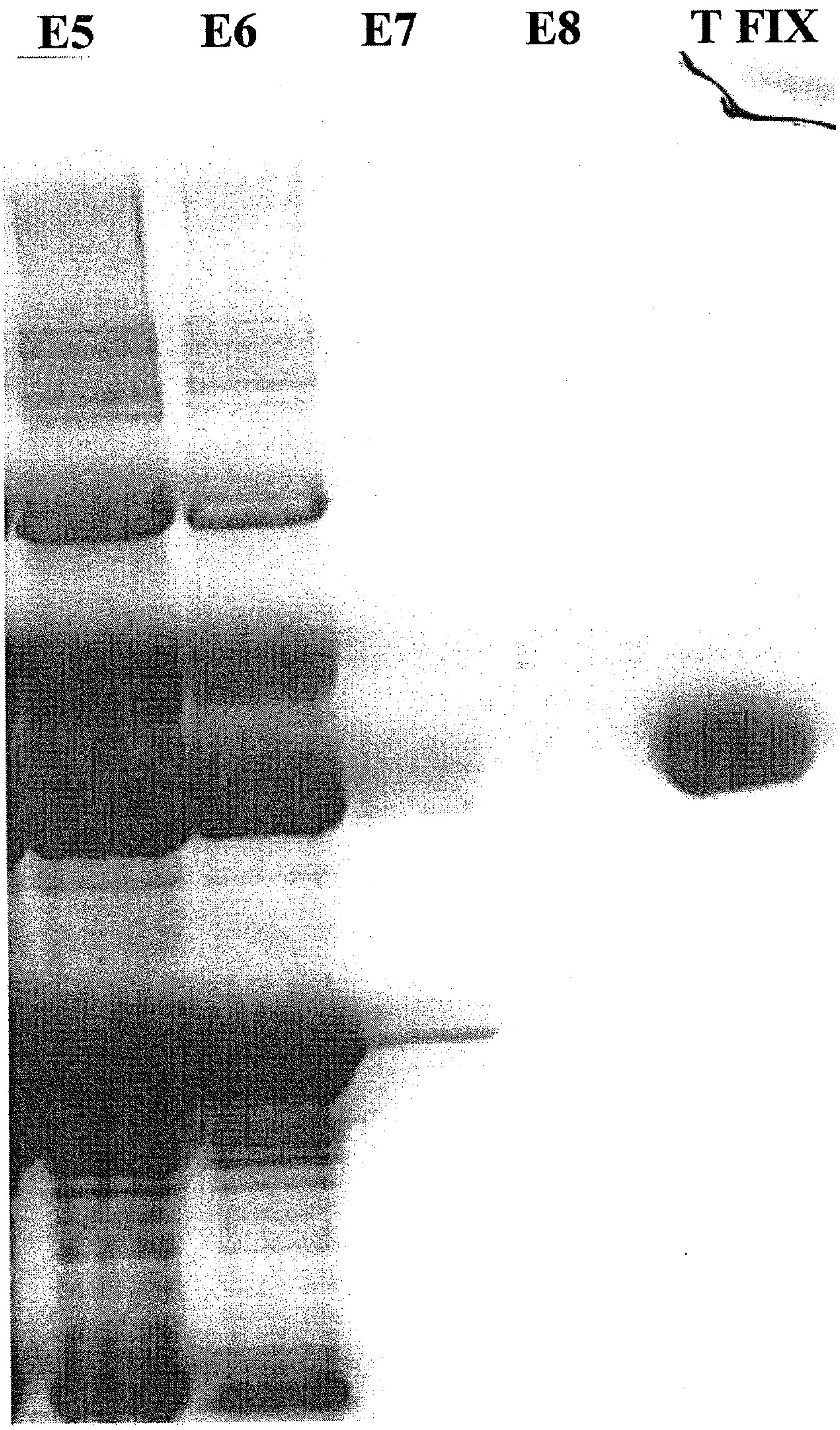


Figure 5

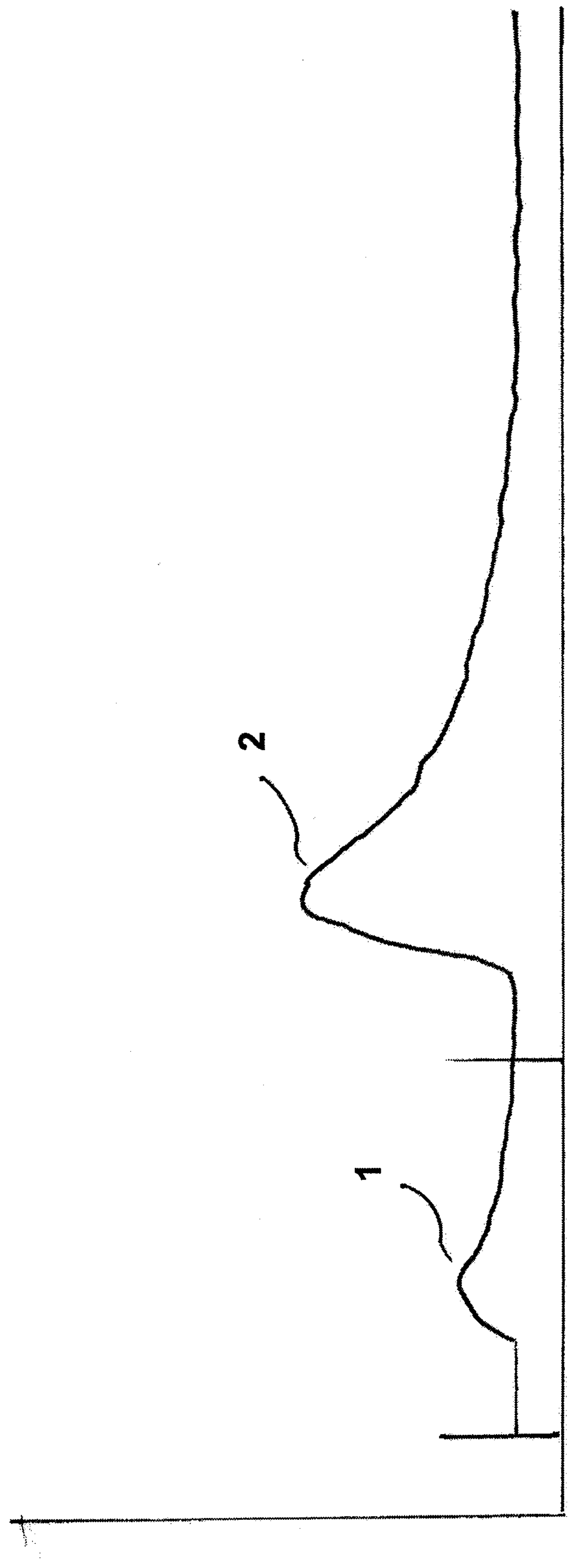


Figure 6

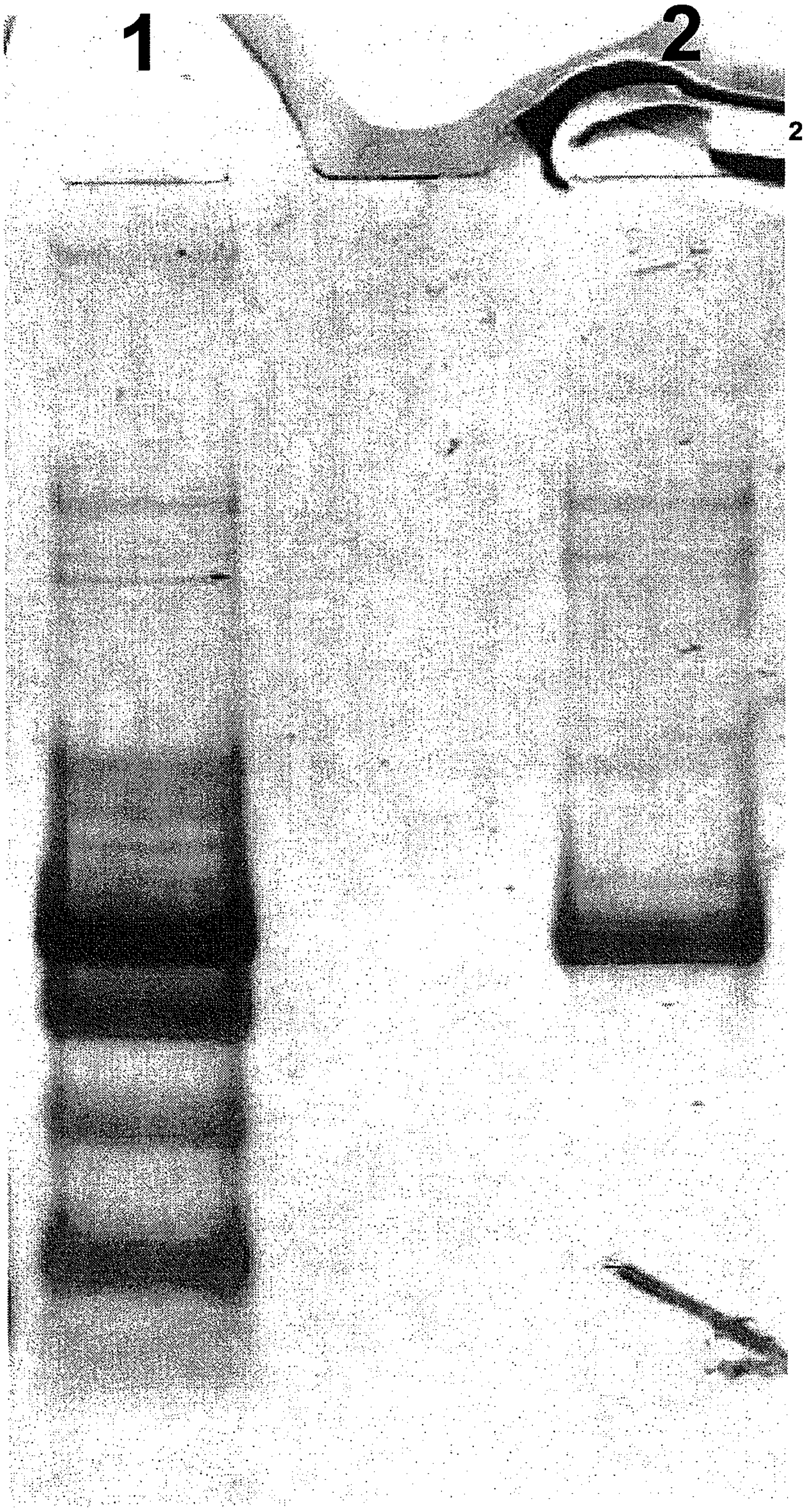


Figure 7

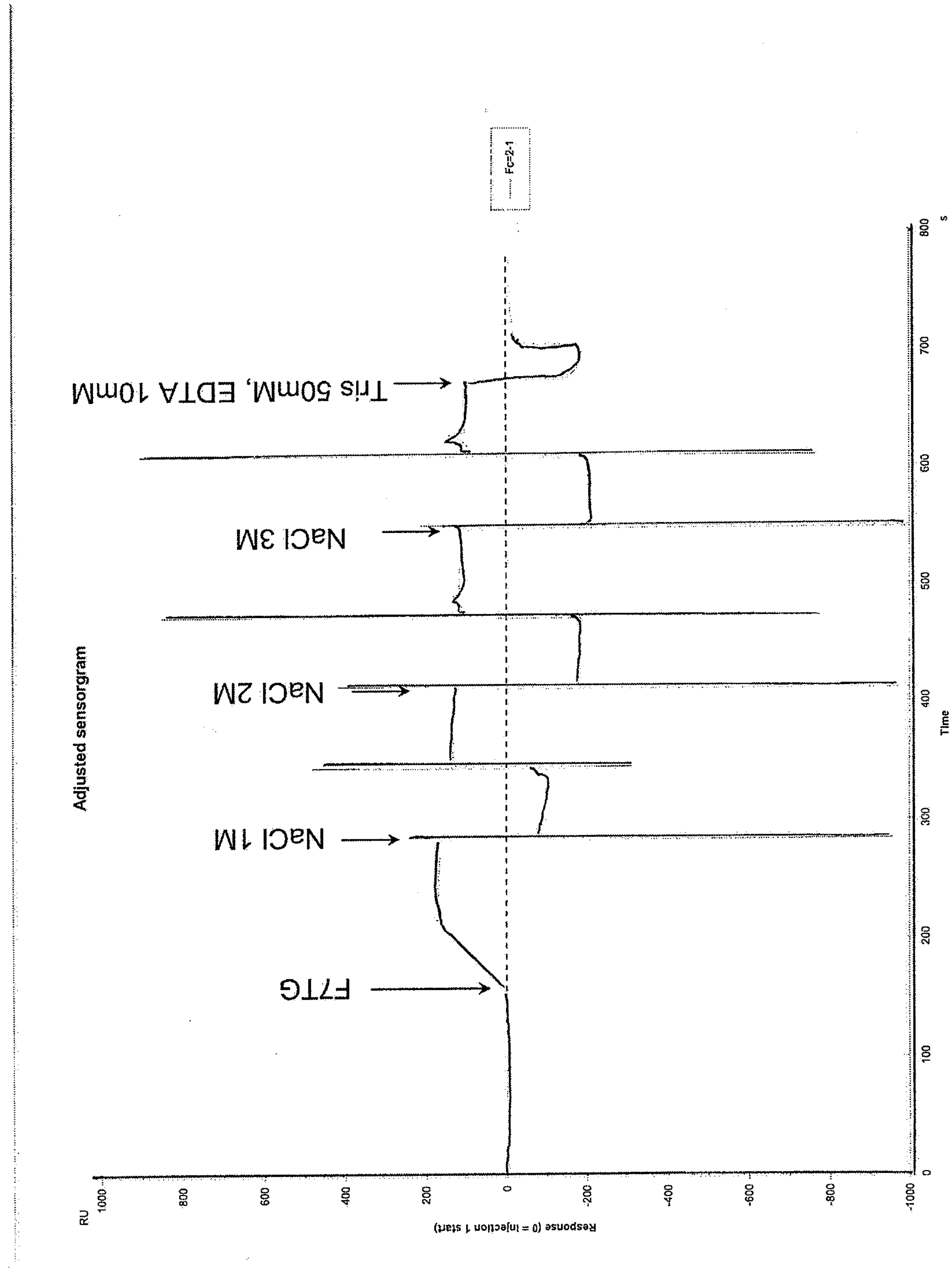
Adjusted sensorgram
RU
Response (0 = Injection 1 start)
Time
s
Fc=2-1
F7TG
NaCl 1M
NaCl 2M
NaCl 3M
Tris 50mM, EDTA 10mM

Figure 8

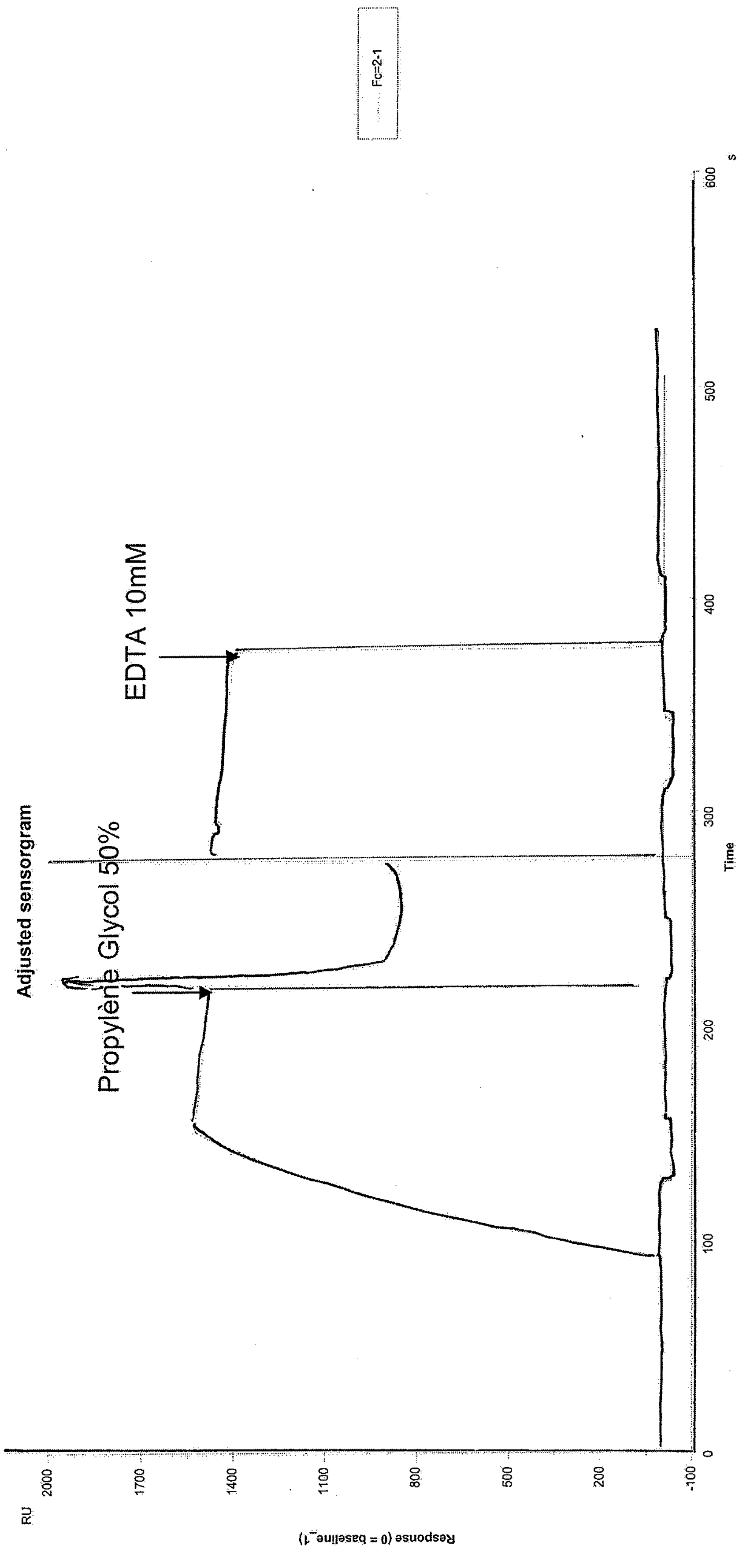
Fc=2-1
Adjusted sensorgram
Propylène Glycol 50%
EDTA 10mM
RU
2000
1700
1400
1100
800
500
200
-100
Response (0 = baseline_1)
0
100
200
300
400
500
600
s
Time

Figure 9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0226932 A
- WO 1991019813 A
- EP 0786469 A
- EP 668931 A
- EP 1695978 A
- EP 1493825 A
- WO 2008150495 A
- EP 1972693 A
- WO 2002096926 A
- US 7312325 B
- EP 0527063 A
- US 7045676 B
- EP 1739170 A
- WO 2008099077 A


### Littérature non-brevet citée dans la description


- **ROMIG et al.** *J. Chromatogr.,* 1999, vol. 731, 275-284
- **LIU et al.** *Journal of molecular récognition,* 2003, vol. 16, 23-27
- **ZHAO et al.** *Anal. Chem,* 2008, vol. 80, 3915-3920
- **MICHAUD et al.** *Anal Chem,* 2003, vol. 76, 1015-1020
- **BRUMBT et al.** *Anal Chem,* 2005, vol. 77, 1993-1998
- **TUERK ; GOLD.** Systematic evolution of ligands by exponential enrichment : RNA ligands to bacteriophage T4 DNA polymerase. *Science,* 1990, vol. 249 (4968), 505-10
- **ELLINGTON ; SZOSTAK.** In vitro selection of RNA molecules that bind specific ligands. *Nature,* 30 Août 1990, vol. 346 (6287), 818-22
- **HERMANSON G.T.** Bioconjugate techniques. Academic Press, 1996, 239-242
- **SAMOSZUK M.K. et al.** *Antibody, Immunoconjugates Radiopharm.,* 1989, vol. 2 (1), 37-46
- **ZHAO et al.** *Anal Chem,* 2008, vol. 80 (19), 7586-7593
- **SUBASH et al.** *Thromb Haemost,* 2006, vol. 95, 767-771
- **HOWARD et al.** *Atherioscl Thromb Vasc Biol,* 2007, vol. 27, 722-727
- **RUSCONI et al.** *Thromb Haemost,* 2000, vol. 84 (5), 841-848
- **LAYZER et al.** *Spring,* 2007, vol. 17, 1-11
- **PASCAL BAILON ; GEORGE K. EHRLICH ; WEN-JIAN FUNG ; WOLFGANG BERTHOLD.** An Overview of Affinity Chromatography. Humana Press, 2000
- **MOHR et al.** Affinity Chromatography: Practical and Theoretical Aspects, Peter Mohr, Klaus Pommerening. CRC Press, 1985